# EUROPÄISCHE PATENTSCHRIFT

(11) **EP 1 334 195 B1**
(45) Veröffentlichungstag und Bekanntmachung des Hinweises auf die Patenterteilung: **21.03.2007**
(21) Anmeldenummer: 01992779.7
(22) Anmeldetag: 29.10.2001
(51) Int. Cl.: C12N 15/19, C07K 14/52, C07K 7/00, C07K 16/24, G01N 33/50, A61K 38/19, A61K 38/04

(54) **ANALOGA, AGONISTEN, ANTAGONISTEN UND VARIANTEN DER OXIDOREDUKTASE-ENZYMAKTIVITÄT DES MAKROPHAGEN-MIGRATIONS-INHIBITIONS-FAKTORS (MIF) ALS IMMUNMODULATOREN, THERAPEUTIKA, DIAGNOSTIKA UND SCREENING-AGENZIEN BEI INFLAMMATORISCHEN UND IMMUNERKRANKUNGEN**
ANALOGUES, AGONISTS, ANTAGONISTS AND VARIANTS OF THE OXIDOREDUCTASE ENZYME ACTIVITY OF THE MACROPHAGE-MIGRATION INHIBITION FACTOR (MIF) AS IMMUNOMODULATORS, MEDICAMENTS, DIAGNOSTICS AND SCREENING AGENTS IN INFLAMMATORY AND IMMUNE DISEASES
ANALOGUES, AGONISTES, ANTAGONISTES ET VARIANTES DE L'ACTIVITE ENZYMATIQUE D'OXYDOREDUCTASE DU FACTEUR D'INHIBITION DE LA MIGRATION DE MACROPHAGES (MIF) EN TANT QU'IMMUNOMODULATEURS, AGENTS THERAPEUTIQUES, DIAGNOSTIQUES ET DE CRIBLAGE DANS LE TRAITEMENT DE MALADIES INFLAMMATOIRES ET IMMUNITAIRES

(30) Priorität: 02.11.2000 DE 10054303
(43) Veröffentlichungstag der Anmeldung: 13.08.2003
(73) Patentinhaber: Fraunhofer-Gesellschaft zur Förderung der angewandten Forschung e.V., 80686 München (DE)
(72) Erfinder: BERNHAGEN, Jürgen, 52066 Aachen (DE); BRUNNER, Herwig, 70569 Stuttgart (DE); KAPURNIOTU, Afroditi, 52066 Aachen (DE); KLEEMANN, Robert, 89584 Ehingen (DE); MISCHKE, Ralf, 67063 Ludwigshafen (DE)
(86) Internationale Anmeldenummer: PCT/EP2001/012476
(87) Internationale Veröffentlichungsnummer: WO 2002/036774

(56) Entgegenhaltungen:
- WO-A-01/38566
- WO-A-97/00959
- BENDRAT KLAUS ET AL: "Biochemical and mutational investigations of the enzymatic activity of macrophage migration inhibitory factor." BIOCHEMISTRY, Bd. 36, Nr. 49, 9. Dezember 1997 (1997-12-09), Seiten 15356-15362, XP002200241 ISSN: 0006-2960
- KLEEMANN ROBERT ET AL: "Characterization of catalytic centre mutants of macrophage migration inhibitory factor (MIF) and comparison to Cys81Ser MIF." EUROPEAN JOURNAL OF BIOCHEMISTRY, Bd. 261, Nr. 3, Mai 1999 (1999-05), Seiten 753-766, XP002208091 ISSN: 0014-2956
- KLEEMANN ROBERT ET AL: "Disulfide analysis reveals a role for macrophage migration inhibitory factor (MIF) as thiol-protein oxidoreductase." JOURNAL OF MOLECULAR BIOLOGY, Bd. 280, Nr. 1, 3. Juli 1998 (1998-07-03), Seiten 85-102, XP002208092 ISSN: 0022-2836
- RIVIER J E ET AL: "GONADOTROPIN-RELEASING HORMONE ANTAGONISTS: NOVEL MEMBERS OF THE AZALINE B FAMILY" JOURNAL OF MEDICINAL CHEMISTRY, AMERICAN CHEMICAL SOCIETY. WASHINGTON, US, Bd. 38, Nr. 14, 1995, Seiten 2649-2662, XP002071775 ISSN: 0022-2623
- NGUYEN,M.T. ET AL.: "A 16-residue peptide fragment of macrophage migration inhibitory factor, MIF-(50-65), exhibits redox activity and has MIF-like biological functions" JOURNAL OF BIOLOGICAL CHEMISTRY, Bd. 278, Nr. 1, 6. September 2002 (2002-09-06), Seite 33654,
- SUN,C. ET AL.: "Macrophage migration inhibitory factor: An intracellular inhibitor of angiotensin II-induced increases in neuronal activity." JOURNAL OF NEUROSCIENCE, Bd. 24, Nr. 44, 3. November 2004 (2004-11-03), Seite 9944,

### SEQUENZPROTOKOLL

<110> Fraunhofer Gesellschaft zur Forderung der ... e.V.
<120> Analoga, Agonisten, Antagonisten...
<130> 15348
<140>
   <141>
<160> 37
<170> PatentIn Ver. 2.1
<210> 1
   <211> 115
   <212> PRT
   <213> Homo sapiens
<400> 1
   Met Pro Met Phe Ile Val Asn Thr Asn Val Pro Arg Ala Ser Val Pro
   1 5 10 15
   Asp Gly Phe Leu Ser Glu Leu Thr Gln Gln Leu Ala Gln Ala Thr Gly
   20 25 30
   Lys Pro Pro Gln Tyr Ile Ala Val His Val Val Pro Asp Gln Leu Met
   35 40 45
   Pro Phe Gly Gly Ser Ser Glu Pro Cys Ala Leu Cys Ser Leu His Ser
   50 55 60
   Ile Gly Lys Ile Gly Gly Ala Gln Asn Arg Ser Tyr Ser Lys Leu Leu
   65 70 75 80
   Cys Gly Leu Leu Ala Glu Arg Leu Arg Ile Ser Pro Asp Arg Val Tyr
   85 90 95
   Ile Asn Tyr Tyr Asp Met Asn Ala Ala Asn Val Gly Trp Asn Asn Ser
   100 105 110
   Thr Phe Ala
   115
<210> 2
   <211> 115
   <212> PRT
   <213> Homo sapiens
<400> 2
   Met Pro Met Phe Ile Val Asn Thr Asn Val Pro Arg Ala Ser Val Pro
   1 5 10 15
   Asp Gly Phe Leu Ser Glu Leu Thr Gln Gln Leu Ala Gln Ala Thr Gly
   20 25 30
   Lys Pro Pro Gln Tyr Ile Ala Val His Val Val Pro Asp Gln Leu Met
   35 40 45
   Pro Phe Gly Gly Ser Ser Glu Pro Cys Ala Leu Cys Ser Leu His Ser
   50 55 60
   Ile Gly Lys Ile Gly Gly Ala Gln Asn Arg Ser Tyr Ser Lys Leu Leu
   65 70 75 80
   Cys Gly Leu Leu Ala Glu Arg Leu Arg Ile Ser Pro Asp Arg Val Tyr
   85 90 95
   Ile Asn Tyr Tyr Asp Met Asn Ala Ala Asn Val Pro Trp Asn Asn Ser
   100 105 110
   Thr Phe Ala
   115
<210> 3
   <211> 115
   <212> PRT
   <213> Homo sapiens
<400> 3
   Met Pro Met Phe Ile Val Asn Thr Asn Val Pro Arg Ala Ser Val Pro
   1 5 10 15
   Asp Gly Phe Leu Ser Glu Leu Thr Gln Gln Leu Ala Gln Ala Thr Gly
   20 25 30
   Lys Pro Pro Gln Tyr Ile Ala Val His Val Val Pro Asp Gln Leu Met
   35 40 45
   Ala Phe Gly Gly Ser Ser Glu Pro Cys Ala Leu Cys Ser Leu His Ser
   50 55 60
   Ile Gly Lys Ile Gly Gly Ala Gln Asn Arg Ser Tyr Ser Lys Leu Leu
   65 70 75 80
   Cys Gly Leu Leu Ala Glu Arg Leu Arg Ile Ser Pro Asp Arg Val Tyr
   85 90 95
   Ile Asn Tyr Tyr Asp Met Asn Ala Ala Asn Val Pro Trp Asn Asn Ser
   100 105 110
   Thr Phe Ala
   115
<210> 4
   <211> 115
   <212> PRT
   <213> Homo sapiens
<400> 4
   Met Pro Met Phe Ile Val Asn Thr Asn Val Pro Arg Ala Ser Val Pro
   1               5                   10                  15
   Asp Gly Phe Leu Ser Glu Leu Thr Gln Gln Leu Ala Gln Ala Thr Gly
               20                  25                  30
   Lys Pro Pro Gln Tyr Ile Ala Pro His Val Val Pro Asp Gln Leu Met
           35                  40                  45
   Pro Phe Gly Gly Ser Ser Glu Pro Cys Ala Leu Cys Ser Leu His Ser
       50                  55                  60
   Ile Gly Lys Ile Gly Gly Ala Gln Asn Arg Ser Tyr Ser Lys Leu Leu
   65                  70                  75                  80
   Cys Gly Leu Leu Ala Glu Arg Leu Arg Ile Ser Pro Asp Arg Val Tyr
                   85                  90                  95
   Ile Asn Tyr Tyr Asp Met Asn Ala Ala Asn Val Gly Trp Asn Asn Ser
               100                 105                 110
   Thr Phe Ala
           115
<210> 5
   <211> 115
   <212> PRT
   <213> Homo sapiens
<400> 5
   Met Pro Met Phe Ile Val Asn Thr Asn Val Pro Arg Ala Ser Val Pro
   1 5 10 15
   Asp Gly Phe Leu Ser Glu Leu Thr Gln Gln Leu Ala Gln Ala Thr Gly
   20 25 30
   Lys Pro Pro Gln Tyr Ile Ala Val His Val Val Pro Asp Gln Leu Met
   35 40 45
   Ala Phe Gly Gly Ser Ser Glu Pro Cys Ala Leu Cys Ser Leu His Ser
   50 55 60
   Ile Gly Lys Ile Gly Gly Ala Gln Asn Arg Ser Tyr Ser Lys Leu Leu
   65 70 75 80
   Cys Gly Leu Leu Ala Glu Arg Leu Arg Ile Ser Pro Asp Arg Val Tyr
   85 90 95
   Pro Asn Tyr Tyr Asp Met Asn Ala Ala Asn Val Pro Trp Asn Asn Ser
   100 105 110
   Thr Phe Ala
   115
<210> 6
   <211> 115
   <212> PRT
   <213> Homo sapiens
<220>
   <221> PEPTIDE
   <222> (41)
   <223> Xaa= Ala, Gly oder Ser
<220>
   <221> PEPTIDE
   <222> (46)
   <223> Xaa=Ala, Gly oder Ser
<400> 6
   Met Pro Met Phe Ile Val Asn Thr Asn Val Pro Arg Ala Ser Val Pro
   1 5 10 15
   Asp Gly Phe Leu Ser Glu Leu Thr Gln Gln Leu Ala Gln Ala Thr Gly
   20 25 30
   Lys Pro Pro Gln Tyr Ile Ala Val Xaa Val Val Pro Asp Xaa Leu Met
   35 40 45
   Ala Phe Gly Gly Ser Ser Glu Pro Cys Ala Leu Cys Ser Leu His Ser
   50 55 60
   Ile Gly Lys Ile Gly Gly Ala Gln Asn Arg Ser Tyr Ser Lys Leu Leu
   65 70 75 80
   Cys Gly Leu Leu Ala Glu Arg Leu Arg Ile Ser Pro Asp Arg Val Tyr
   85 90 95
   Ile Asn Tyr Tyr Asp Met Asn Ala Ala Asn Val Gly Trp Asn Asn Ser
   100 105 110
   Thr Phe Ala
   115
<210> 7
   <211> 115
   <212> PRT
   <213> Homo sapiens
<220>
   <221> PEPTIDE
   <222> (49)
   <223> Xaa= N alpha-Ala
<400> 7
   Met Pro Met Phe Ile Val Asn Thr Asn Val Pro Arg Ala Ser Val Pro
   1 5 10 15
   Asp Gly Phe Leu Ser Glu Leu Thr Gln Gln Leu Ala Gln Ala Thr Gly
                   20                  25                  30
   Lys Pro Pro Gln Tyr Ile Ala Val His Val Val Pro Asp Gln Leu Met
               35                  40                  45
   Xaa Phe Gly Gly Ser Ser Glu Pro Cys Ala Leu Cys Ser Leu His Ser
           50                  55                  60
   Ile Gly Lys Ile Gly Gly Ala Gln Asn Arg Ser Tyr Ser Lys Leu Leu
   65                  70                  75                  80
   Cys Gly Leu Leu Ala Glu Arg Leu Arg Ile Ser Pro Asp Arg Val Tyr
                   85                  90                  95
   Ile Asn Tyr Tyr Asp Met Asn Ala Ala Asn Val Gly Trp Asn Asn Ser
               100                 105                 110
   Thr Phe Ala
           115
<210> 8
   <211> 115
   <212> PRT
   <213> Homo sapiens
<220>
   <221> PEPTIDE
   <222> (108)
   <223> Xaa=N alpha-Gly
<220>
   <221> PEPTIDE
   <222> (49)
   <223> Xaa= N alpha -Ala
<400> 8
   Met Pro Met Phe Ile Val Asn Thr Asn Val Pro Arg Ala Ser Val Pro
   1 5 10 15
   Asp Gly Phe Leu Ser Glu Leu Thr Gln Gln Leu Ala Gln Ala Thr Gly
   20 25 30
   Lys Pro Pro Gln Tyr Ile Ala Val His Val Val Pro Asp Gln Leu Met
   35 40 45
   Xaa Phe Gly Gly Ser Ser Glu Pro Cys Ala Leu Cys Ser Leu His Ser
   50 55 60
   Ile Gly Lys Ile Gly Gly Ala Gln Asn Arg Ser Tyr Ser Lys Leu Leu
   65 70 75 80
   Cys Gly Leu Leu Ala Glu Arg Leu Arg Ile Ser Pro Asp Arg Val Tyr
   85 90 95
   Ile Asn Tyr Tyr Asp Met Asn Ala Ala Asn Val Xaa Trp Asn Asn Ser
   100 105 110
   Thr Phe Ala
   115
<210> 9
   <211> 115
   <212> PRT
   <213> Homo sapiens
<220>
   <221> PEPTIDE
   <222> (108)
   <223> Xaa= N alpha-Gly
<400> 9
   Met Pro Met Phe Ile Val Asn Thr Asn Val Pro Arg Ala Ser Val Pro
   1 5 10 15
   Asp Gly Phe Leu Ser Glu Leu Thr Gln Gln Leu Ala Gln Ala Thr Gly
   20 25 30
   Lys Pro Pro Gln Tyr Ile Ala Val His Val Val Pro Asp Gln Leu Met
   35 40 45
   Ala Phe Gly Gly Ser Ser Glu Pro Cys Ala Leu Cys Ser Leu His Ser
   50 55 60
   Ile Gly Lys Ile Gly Gly Ala Gln Asn Arg Ser Tyr Ser Lys Leu Leu
   65 70 75 80
   Cys Gly Leu Leu Ala Glu Arg Leu Arg Ile Ser Pro Asp Arg Val Tyr
   85 90 95
   Ile Asn Tyr Tyr Asp Met Asn Ala Ala Asn Val Xaa Trp Asn Asn Ser
   100 105 110
   Thr Phe Ala
   115
<210> 10
   <211> 115
   <212> PRT
   <213> Homo sapiens
<220>
   <221> PEPTIDE
   <222> (40)
   <223> Xaa=N alpha-Val
<220>
   <221> PEPTIDE
   <222> (49)
   <223> Xaa=N alpha-Ala
<400> 10
   Met Pro Met Phe Ile Val Asn Thr Asn Val Pro Arg Ala Ser Val Pro
   1 5 10 15
   Asp Gly Phe Leu Ser Glu Leu Thr Gln Gln Leu Ala Gln Ala Thr Gly
   20 25 30
   Lys Pro Pro Gln Tyr Ile Ala Xaa His Val Val Pro Asp Gln Leu Met
   35 40 45
   Xaa Phe Gly Gly Ser Ser Glu Pro Cys Ala Leu Cys Ser Leu His Ser
   50 55 60
   Ile Gly Lys Ile Gly Gly Ala Gln Asn Arg Ser Tyr Ser Lys Leu Leu
   65 70 75 80
   Cys Gly Leu Leu Ala Glu Arg Leu Arg Ile Ser Pro Asp Arg Val Tyr
   85 90 95
   Ile Asn Tyr Tyr Asp Met Asn Ala Ala Asn Val Gly Trp Asn Asn Ser
   100 105 110
   Thr Phe Ala
   115
<210> 11
   <211> 115
   <212> PRT
   <213> Homo sapiens
<220>
   <221> PEPTIDE
   <222> (97)
   <223> Xaa=N alpha-Ile
<220>
   <221> PEPTIDE
   <222> (108)
   <223> Xaa=N alpha-Gly
<400> 11
   Met Pro Met Phe Ile Val Asn Thr Asn Val Pro Arg Ala Ser Val Pro
   1 5 10 15
   Asp Gly Phe Leu Ser Glu Leu Thr Gln Gln Leu Ala Gln Ala Thr Gly
   20 25 30
   Lys Pro Pro Gln Tyr Ile Ala Val His Val Val Pro Asp Gln Leu Met
   35 40 45
   Ala Phe Gly Gly Ser Ser Glu Pro Cys Ala Leu Cys Ser Leu His Ser
   50 55 60
   Ile Gly Lys Ile Gly Gly Ala Gln Asn Arg Ser Tyr Ser Lys Leu Leu
   65 70 75 80
   Cys Gly Leu Leu Ala Glu Arg Leu Arg Ile Ser Pro Asp Arg Val Tyr
   85 90 95
   Xaa Asn Tyr Tyr Asp Met Asn Ala Ala Asn Val Xaa Trp Asn Asn Ser
   100 105 110
   Thr Phe Ala
   115
<210> 12
   <211> 115
   <212> PRT
   <213> Homo sapiens
<220>
   <221> PEPTIDE
   <222> (57)
   <223> Xaa= eine unnatürliche Aminosäure mit struktureller Seitenkettenähnlichkeit zu Vinylpyridin oder DTNB
<220>
   <221> PEPTIDE
   <222> (60)
   <223> Xaa= eine unnatürliche Aminosäure mit struktureller Seitenkettenähnlichkeit zu Vinylpyridin oder DTNB
<220>
   <221> PEPTIDE
   <222> (81)
   <223> Xaa=eine unnatürliche Aminosäure mit struktureller Seitenkettenähnlichkeit zu Vinylpyridin oder DTNB
<400> 12
   Met Pro Met Phe Ile Val Asn Thr Asn Val Pro Arg Ala Ser Val Pro
   1               5                   10                  15
   Asp Gly Phe Leu Ser Glu Leu Thr Gln Gln Leu Ala Gln Ala Thr Gly
               20                  25                  30
   Lys Pro Pro Gln Tyr Ile Ala Val His Val Val Pro Asp Gln Leu Met
           35                  40                  45
   Ala Phe Gly Gly Ser Ser Glu Pro Xaa Ala Leu Xaa Ser Leu His Ser
       50                  55                  60
   Ile Gly Lys Ile Gly Gly Ala Gln Asn Arg Ser Tyr Ser Lys Leu Leu
   65                  70                  75                  80
   Xaa Gly Leu Leu Ala Glu Arg Leu Arg Ile Ser Pro Asp Arg Val Tyr
                   85                  90                  95
   Ile Asn Tyr Tyr Asp Met Asn Ala Ala Asn Val Gly Trp Asn Asn Ser
               100                 105                 110
   Thr Phe Ala
           115
<210> 13
   <211> 115
   <212> PRT
   <213> Homo sapiens
<220>
   <221> PEPTIDE
   <222> (56)
   <223> Xaa= Lys, Orn, Dab, Dap oder Glu
<220>
   <221> PEPTIDE
   <222> (61)
   <223> Xaa=Asp, Lys, Orn, Dab, Dap oder Glu
<400> 13
   Met Pro Met Phe Ile Val Asn Thr Asn Val Pro Arg Ala Ser Val Pro
   1 5 10 15
   Asp Gly Phe Leu Ser Glu Leu Thr Gln Gln Leu Ala Gln Ala Thr Gly
   20 25 30
   Lys Pro Pro Gln Tyr Ile Ala Val His Val Val Pro Asp Gln Leu Met
   35 40 45
   Ala Phe Gly Gly Ser Ser Glu Xaa Cys Ala Leu Cys Xaa Leu His Ser
   50 55 60
   Ile Gly Lys Ile Gly Gly Ala Gln Asn Arg Ser Tyr Ser Lys Leu Leu
   65 70 75 80
   Cys Gly Leu Leu Ala Glu Arg Leu Arg Ile Ser Pro Asp Arg Val Tyr
   85 90 95
   Ile Asn Tyr Tyr Asp Met Asn Ala Ala Asn Val Gly Trp Asn Asn Ser
   100 105 110
   Thr Phe Ala
   115
<210> 14
   <211> 115
   <212> PRT
   <213> Homo sapiens
<220>
   <221> PEPTIDE
   <222> (55)
   <223> Xaa=Lys, Asp, Orn, Dab, Dap oder Glu
<220>
   <221> PEPTIDE
   <222> (62)
   <223> Xaa= Asp, Lys, Orn, Dab, Dap oder Glu
<400> 14
   Met Pro Met Phe Ile Val Asn Thr Asn Val Pro Arg Ala Ser Val Pro
   1 5 10 15
   Asp Gly Phe Leu Ser Glu Leu Thr Gln Gln Leu Ala Gln Ala Thr Gly
   20 25 30
   Lys Pro Pro Gln Tyr Ile Ala Val His Val Val Pro Asp Gln Leu Met
   35 40 45
   Ala Phe Gly Gly Ser Ser Xaa Pro Cys Ala Leu Cys Ser Xaa His Ser
   50 55 60
   Ile Gly Lys Ile Gly Gly Ala Gln Asn Arg Ser Tyr Ser Lys Leu Leu
   65 70 75 80
   Cys Gly Leu Leu Ala Glu Arg Leu Arg Ile Ser Pro Asp Arg Val Tyr
   85 90 95
   Ile Asn Tyr Tyr Asp Met Asn Ala Ala Asn Val Gly Trp Asn Asn Ser
   100 105 110
   Thr Phe Ala
   115
<210> 15
   <211> 115
   <212> PRT
   <213> Homo sapiens
<220>
   <221> PEPTIDE
   <222> (56)
   <223> Xaa=Lys, Asp, Orn, Dab, Dap oder Glu
<220>
   <221> PEPTIDE
   <222> (57)
   <223> Xaa=Ser, Ala oder Gly
<220>
   <221> PEPTIDE
   <222> (60)
   <223> Xaa=Ser, Ala oder Gly
<220>
   <221> PEPTIDE
   <222> (61)
   <223> Xaa=Asp, Lys, Orn, Dab, Dap oder Glu
<400> 15
   Met Pro Met Phe Ile Val Asn Thr Asn Val Pro Arg Ala Ser Val Pro
   1 5 10 15
   Asp Gly Phe Leu Ser Glu Leu Thr Gln Gln Leu Ala Gln Ala Thr Gly
   20 25 30
   Lys Pro Pro Gln Tyr Ile Ala Val His Val Val Pro Asp Gln Leu Met
   35 40 45
   Ala Phe Gly Gly Ser Ser Glu Xaa Xaa Ala Leu Xaa Xaa Leu His Ser
   50 55 60
   Ile Gly Lys Ile Gly Gly Ala Gln Asn Arg Ser Tyr Ser Lys Leu Leu
   65 70 75 80
   Cys Gly Leu Leu Ala Glu Arg Leu Arg Ile Ser Pro Asp Arg Val Tyr
   85 90 95
   Ile Asn Tyr Tyr Asp Met Asn Ala Ala Asn Val Gly Trp Asn Asn Ser
   100 105 110
   Thr Phe Ala
   115
<210> 16
   <211> 115
   <212> PRT
   <213> Homo sapiens
<220>
   <221> PEPTIDE
   <222> (55)
   <223> Xaa=Lys, Asp, Orn, Dab, Dap oder Glu
<220>
   <221> PEPTIDE
   <222> (57)
   <223> Xaa=Ser, Ala oder Gly
<220>
   <221> PEPTIDE
   <222> (60)
   <223> Xaa=Ser, Ala oder Gly
<220>
   <221> PEPTIDE
   <222> (62)
   <223> Xaa=Asp, Lys, Orn, Dab, Dap oder Glu
<400> 16
   Met Pro Met Phe Ile Val Asn Thr Asn Val Pro Arg Ala Ser Val Pro
   1 5 10 15
   Asp Gly Phe Leu Ser Glu Leu Thr Gln Gln Leu Ala Gln Ala Thr Gly
   20 25 30
   Lys Pro Pro Gln Tyr Ile Ala Val His Val Val Pro Asp Gln Leu Met
   35 40 45
   Ala Phe Gly Gly Ser Ser Xaa Pro Xaa Ala Leu Xaa Ser Xaa His Ser
   50 55 60
   Ile Gly Lys Ile Gly Gly Ala Gln Asn Arg Ser Tyr Ser Lys Leu Leu
   65 70 75 80
   Cys Gly Leu Leu Ala Glu Arg Leu Arg Ile Ser Pro Asp Arg Val Tyr
   85 90 95
   Ile Asn Tyr Tyr Asp Met Asn Ala Ala Asn Val Gly Trp Asn Asn Ser
   100 105 110
   Thr Phe Ala
   115
<210> 17
   <211> 16
   <212> PRT
   <213> Homo sapiens
<400> 17
   Phe Gly Gly Ser Ser Glu Pro Cys Ala Leu Cys Ser Leu His Ser Ile
   1 5 10 15
<210> 18
   <211> 16
   <212> PRT
   <213> Homo sapiens
<400> 18
   Gly Gly Ser Ser Glu Pro Cys Ala Leu Cys Ser Leu His Ser Ile Gly
   1 5 10 15
<210> 19
   <211> 11
   <212> PRT
   <213> Homo sapiens
<400> 19
   Glu Pro Cys Ala Leu Cys Ser Leu His Ser Ile
   1 5 10
<210> 20
   <211> 12
   <212> PRT
   <213> Homo sapiens
<400> 20
   Ser Glu Pro Cys Ala Leu Cys Ser Leu His Ser Ile
   1 5 10
<210> 21
   <211> 13
   <212> PRT
   <213> Homo sapiens
<400> 21
   Ser Ser Glu Pro Cys Ala Leu Cys Ser Leu His Ser Ile
   1 5 10
<210> 22
   <211> 14
   <212> PRT
   <213> Homo sapiens
<400> 22
   Gly Ser Ser Glu Pro Cys Ala Leu Cys Ser Leu His Ser Ile
   1 5 10
<210> 23
   <211> 15
   <212> PRT
   <213> Homo sapiens
<400> 23
   Gly Gly Ser Ser Glu Pro Cys Ala Leu Cys Ser Leu His Ser Ile
   1 5 10 15
<210> 24
   <211> 16
   <212> PRT
   <213> Homo sapiens
<220>
   <221> PEPTIDE
   <222> (8)
   <223> Xaa= Ser, Ala oder Gly
<220>
   <221> PEPTIDE
   <222> (11)
   <223> Xaa= Ser, Ala oder Gly
<400> 24
   Phe Gly Gly Ser Ser Glu Pro Xaa Ala Leu Xaa Ser Leu His Ser Ile
   1               5                   10                  15
<210> 25
   <211> 115
   <212> PRT
   <213> Homo sapiens
<400> 25
   Met Pro Met Phe Ile Val Asn Thr Asn Val Pro Arg Ala Ser Val Pro
   1               5                   10                  15
   Asp Gly Phe Leu Ser Glu Leu Thr Gln Gln Leu Ala Gln Ala Thr Gly
               20                  25                  30
   Lys Pro Pro Gln Tyr Ile Ala Val His Val Val Pro Asp Gln Leu Met
           35                  40                  45
   Ala Phe Gly Gly Ser Ser Glu Pro Ser Ala Leu Ser Ser Leu His Ser
       50                  55                  60
   Ile Gly Lys Ile Gly Gly Ala Gln Asn Arg Ser Tyr Ser Lys Leu Leu
   65                  70                  75                  80
   Ser Gly Leu Leu Ala Glu Arg Leu Arg Ile Ser Pro Asp Arg Val Tyr
                   85                  90                  95
   Ile Asn Tyr Tyr Asp Met Asn Ala Ala Asn Val Gly Trp Asn Asn Ser
               100                 105                 110
   Thr Phe Ala
           115
<210> 26
   <211> 115
   <212> PRT
   <213> Homo sapiens
<400> 26
   Met Pro Met Phe Ile Val Asn Thr Asn Val Pro Arg Ala Ser Val Pro
   1 5 10 15
   Asp Gly Phe Leu Ser Glu Leu Thr Gln Gln Leu Ala Gln Ala Thr Gly
   20 25 30
   Lys Pro Pro Gln Tyr Ile Ala Val His Val Val Pro Asp Gln Leu Met
   35 40 45
   Ala Phe Gly Gly Ser Ser Glu Pro Ala Ala Leu Ala Ser Leu His Ser
   50 55 60
   Ile Gly Lys Ile Gly Gly Ala Gln Asn Arg Ser Tyr Ser Lys Leu Leu
   65 70 75 80
   Ala Gly Leu Leu Ala Glu Arg Leu Arg Ile Ser Pro Asp Arg Val Tyr
   85 90 95
   Ile Asn Tyr Tyr Asp Met Asn Ala Ala Asn Val Gly Trp Asn Asn Ser
   100 105 110
   Thr Phe Ala
   115
<210> 27
   <211> 115
   <212> PRT
   <213> Homo sapiens
<400> 27
   Met Pro Met Phe Ile Val Asn Thr Asn Val Pro Arg Ala Ser Val Pro
   1 5 10 15
   Asp Gly Phe Leu Ser Glu Leu Thr Gln Gln Leu Ala Gln Ala Thr Gly
   20 25 30
   Lys Pro Pro Gln Tyr Ile Ala Val His Val Val Pro Asp Gln Leu Met
   35 40 45
   Ala Phe Gly Gly Ser Ser Glu Pro Phe Ala Leu Phe Ser Leu His Ser
   50 55 60
   Ile Gly Lys Ile Gly Gly Ala Gln Asn Arg Ser Tyr Ser Lys Leu Leu
   65 70 75 80
   Phe Gly Leu Leu Ala Glu Arg Leu Arg Ile Ser Pro Asp Arg Val Tyr
   85 90 95
   Ile Asn Tyr Tyr Asp Met Asn Ala Ala Asn Val Gly Trp Asn Asn Ser
   100 105 110
   Thr Phe Ala
   115
<210> 28
   <211> 115
   <212> PRT
   <213> Homo sapiens
<400> 28
   Met Pro Met Phe Ile Val Asn Thr Asn Val Pro Arg Ala Ser Val Pro
   1 5 10 15
   Asp Gly Phe Leu Ser Glu Leu Thr Gln Gln Leu Ala Gln Ala Thr Gly
   20 25 30
   Lys Pro Pro Gln Tyr Ile Ala Val His Val Val Pro Asp Gln Leu Met
   35 40 45
   Ala Phe Gly Gly Ser Ser Glu Pro Trp Ala Leu Trp Ser Leu His Ser
   50 55 60
   Ile Gly Lys Ile Gly Gly Ala Gln Asn Arg Ser Tyr Ser Lys Leu Leu
   65 70 75 80
   Trp Gly Leu Leu Ala Glu Arg Leu Arg Ile Ser Pro Asp Arg Val Tyr
   85 90 95
   Ile Asn Tyr Tyr Asp Met Asn Ala Ala Asn Val Gly Trp Asn Asn Ser
   100 105 110
   Thr Phe Ala
   115
<210> 29
   <211> 115
   <212> PRT
   <213> Homo sapiens
<400> 29
   Met Pro Met Phe Ile Val Asn Thr Asn Val Pro Arg Ala Ser Val Pro
   1 5 10 15
   Asp Gly Phe Leu Ser Glu Leu Thr Gln Gln Leu Ala Gln Ala Thr Gly
   20 25 30
   Lys Pro Pro Gln Tyr Ile Ala Val His Val Val Pro Asp Gln Leu Met
   35 40 45
   Ala Phe Gly Gly Ser Ser Glu Pro Tyr Ala Leu Tyr Ser Leu His Ser
   50 55 60
   Ile Gly Lys Ile Gly Gly Ala Gln Asn Arg Ser Tyr Ser Lys Leu Leu
   65 70 75 80
   Tyr Gly Leu Leu Ala Glu Arg Leu Arg Ile Ser Pro Asp Arg Val Tyr
   85 90 95
   Ile Asn Tyr Tyr Asp Met Asn Ala Ala Asn Val Gly Trp Asn Asn Ser
   100 105 110
   Thr Phe Ala
   115
<210> 30
   <211> 115
   <212> PRT
   <213> Homo sapiens
<220>
   <221> PEPTIDE
   <222> (57)
   <223> Xaa= Cys-Rest, der mit einem Alkylierungsrest modifiziert wurde
<220>
   <221> PEPTIDE
   <222> (60)
   <223> Xaa= Cys-Rest, der mit einem Alkylierungsrest modifiziert wurde
<220>
   <221> PEPTIDE
   <222> (81)
   <223> Xaa= Cys-Rest, der mit einem Alkylierungsrest modifiziert wurde
<400> 30
   Met Pro Met Phe Ile Val Asn Thr Asn Val Pro Arg Ala Ser Val Pro
     1               5                  10                  15
   Asp Gly Phe Leu Ser Glu Leu Thr Gln Gln Leu Ala Gln Ala Thr Gly
               20                  25                  30
   Lys Pro Pro Gln Tyr Ile Ala Val His Val Val Pro Asp Gln Leu Met
           35                  40                  45
   Ala Phe Gly Gly Ser Ser Glu Pro Xaa Ala Leu Xaa Ser Leu His Ser
       50                  55                  60
   Ile Gly Lys Ile Gly Gly Ala Gln Asn Arg Ser Tyr Ser Lys Leu Leu
   65                  70                  75                  80
   Xaa Gly Leu Leu Ala Glu Arg Leu Arg Ile Ser Pro Asp Arg Val Tyr
                   85                  90                  95
   Ile Asn Tyr Tyr Asp Met Asn Ala Ala Asn Val Gly Trp Asn Asn Ser
               100                 105                 110
   Thr Phe Ala
           115
<210> 31
   <211> 21
   <212> PRT
   <213> Homo sapiens
<220>
   <221> PEPTIDE
   <222> (9)..(12)
   <223> Xaa steht für kleine Aminosäuren wie Gly oder Aminosäuren, die häufig in beta-Turns anzutreffen sind, oder Aminosäuren, die zur Lactambrückenbildung genutzt werden können, wie Asp, Glu, Lys, Orn us
<400> 31
   Met Pro Met Phe Ile Val Asn Thr Xaa Xaa Xaa Xaa Cys Ala Leu Cys
     1               5                  10                  15
   Ser Leu His Ser Ile
               20
<210> 32
   <211> 22
   <212> PRT
   <213> Homo sapiens
<220>
   <221> PEPTIDE
   <222> (9) .. (13)
   <223> Xaa steht für kleine Aminosäuren wie Gly oder Aminosäuren, die häufig in beta-Turns anzutreffen sind, oder Aminosäuren, die zur Lactambrückenbildung genutzt werden können, wie Asp, Glu, Lys, Orn us
<400> 32
   Met Pro Met Phe Ile Val Asn Thr Xaa Xaa Xaa Xaa Xaa Cys Ala Leu
   1 5 10 15
   Cys Ser Leu His Ser Ile
   20
<210> 33
   <211> 23
   <212> PRT
   <213> Homo sapiens
<220>
   <221> PEPTIDE
   <222> (9)..(14)
   <223> Xaa steht für kleine Aminosäuren wie Gly oder Aminosäuren, die häufig in beta-Turns anzutreffen sind, oder Aminosäuren, die zur Lactambrückenbildung genutzt werden können, wie Asp, Glu, Lys, Orn usw
<400> 33
   Met Pro Met Phe Ile Val Asn Thr Xaa Xaa Xaa Xaa Xaa Xaa Cys Ala
   1 5 10 15
   Leu Cys Ser Leu His Ser Ile
   20
<210> 34
   <211> 22
   <212> PRT
   <213> Homo sapiens
<220>
   <221> PEPTIDE
   <222> (10).. (12)
   <223> Xaa steht für kleine Aminosäuren wie Gly oder Aminosäuren, die häufig in beta-Turns anzutreffen sind, oder Aminosäuren, die zur Lactambrückenbildung genutzt werden können, wie Asp, Glu, Lys, Orn usw.
<400> 34
   Met Pro Met Phe Ile Val Asn Thr Asn Xaa Xaa Xaa Pro Cys Ala Leu
   1 5 10 15
   Cys Ser Leu His Ser Ile
   20
<210> 35
   <211> 23
   <212> PRT
   <213> Homo sapiens
<220>
   <221> PEPTIDE
   <222> (10)..(13)
   <223> Xaa steht für kleine Aminosäuren wie Gly oder Aminosäuren, die häufig in beta-Turns anzutreffen sind, oder Aminosäuren, die zur Lactambrückenbildung genutzt werden können, wie Asp, Glu, Lys, Orn usw.
<400> 35
   Met Pro Met Phe Ile Val Asn Thr Asn Xaa Xaa Xaa Xaa Pro Cys Ala
    1               5                   10                  15
   Leu Cys Ser Leu His Ser Ile
               20
<210> 36
   <211> 24
   <212> PRT
   <213> Homo sapiens
<220>
   <221> PEPTIDE
   <222> (10) .. (14)
   <223> Xaa steht für kleine Aminosäuren wie Gly oder Aminosäuren, die häufig in beta-Turns anzutreffen sind, oder Aminosäuren, die zur Lactambrückenbildung genutzt werden können, wie Asp, Glu, Lys, Orn usw.
<400> 36
   Met Pro Met Phe Ile Val Asn Thr Asn Xaa Xaa Xaa Xaa Xaa Pro Cys
    1               5                   10                  15
   Ala Leu Cys Ser Leu His Ser Ile
               20
<210> 37
   <211> 25
   <212> PRT
   <213> Homo sapiens
<220>
   <221> PEPTIDE
   <222> (10)..(15)
   <223> Xaa steht für kleine Aminosäuren wie Gly oder Aminosäuren, die häufig in beta-Turns anzutreffen sind, oder Aminosäuren, die zur Lactambrückenbildung genutzt werden können, wie Asp, Glu, Lys, Orn usw.
<400> 37
   Met Pro Met Phe Ile Val Asn Thr Asn Xaa Xaa Xaa Xaa Xaa Xaa Pro
    1               5                   10                  15
   Cys Ala Leu Cys Ser Leu His Ser Ile
               20                  25

### SEQUENZPROTOKOLL

<110> Fraunhofer Gesellschaft zur Forderung der ... e.V.
<120> Analoga, Agonisten, Antagonisten...
<130> 15348
<140>
   <141>
<160> 37
<170> PatentIn Ver. 2.1
<210>1
   <211> 115
   <212> PRT
   <213> Homo sapiens
<400> 1
   Met Pro Met Phe Ile Val Asn Thr Asn Val Pro Arg Ala Ser Val Pro
    1               5                  10                  15
   Asp Gly Phe Leu Ser Glu Leu Thr Gln Gln Leu Ala Gln Ala Thr Gly
               20                  25                  30
   Lys Pro Pro Gln Tyr Ile Ala Val His Val Val Pro Asp Gln Leu Met
           35                  40                  45
   Pro Phe Gly Gly Ser Ser Glu Pro Cys Ala Leu Cys Ser Leu His Ser
       50                  55                  60
   Ile Gly Lys Ile Gly Gly Ala Gln Asn Arg Ser Tyr Ser Lys Leu Leu
   65                  70                  75                  80
   Cys Gly Leu Leu Ala Glu Arg Leu Arg Ile Ser Pro Asp Arg Val Tyr
                   85                  90                  95
   Ile Asn Tyr Tyr Asp Met Asn Ala Ala Asn Val Gly Trp Asn Asn Ser
               100                 105                 110
   Thr Phe Ala
           115
<210> 2
   <211> 115
   <212> PRT
   <213> Homo sapiens
<400> 2
   Met Pro Met Phe Ile Val Asn Thr Asn Val Pro Arg Ala Ser Val Pro
    1               5                  10                  15
   Asp Gly Phe Leu Ser Glu Leu Thr Gln Gln Leu Ala Gln Ala Thr Gly
               20                  25                  30
   Lys Pro Pro Gln Tyr Ile Ala Val His Val Val Pro Asp Gln Leu Met
   35 40 45
   Pro Phe Gly Gly Ser Ser Glu Pro Cys Ala Leu Cys Ser Leu His Ser
   50 55 60
   Ile Gly Lys Ile Gly Gly Ala Gln Asn Arg Ser Tyr Ser Lys Leu Leu
   65 70 75 80
   Cys Gly Leu Leu Ala Glu Arg Leu Arg Ile Ser Pro Asp Arg Val Tyr
   85 90 95
   Ile Asn Tyr Tyr Asp Met Asn Ala Ala Asn Val Pro Trp Asn Asn Ser
   100 105 110
   Thr Phe Ala
   115
<210> 3
   <211> 115
   <212> PRT
   <213> Homo sapiens
<400> 3
   Met Pro Met Phe Ile Val Asn Thr Asn Val Pro Arg Ala Ser Val Pro
   1 5 10 15
   Asp Gly Phe Leu Ser Glu Leu Thr Gln Gln Leu Ala Gln Ala Thr Gly
   20 25 30
   Lys Pro Pro Gln Tyr Ile Ala Val His Val Val Pro Asp Gln Leu Met
   35 40 45
   Ala Phe Gly Gly Ser Ser Glu Pro Cys Ala Leu Cys Ser Leu His Ser
   50 55 60
   Ile Gly Lys Ile Gly Gly Ala Gln Asn Arg Ser Tyr Ser Lys Leu Leu
   65 70 75 80
   Cys Gly Leu Leu Ala Glu Arg Leu Arg Ile Ser Pro Asp Arg Val Tyr
   85 90 95
   Ile Asn Tyr Tyr Asp Met Asn Ala Ala Asn Val Pro Trp Asn Asn Ser
   100 105 110
   Thr Phe Ala
   115
<210> 4
   <211> 115
   <212> PRT
   <213> Homo sapiens
<400> 4
   Met Pro Met Phe Ile Val Asn Thr Asn Val Pro Arg Ala Ser Val Pro
    1               5                  10                  15
   Asp Gly Phe Leu Ser Glu Leu Thr Gln Gln Leu Ala Gln Ala Thr Gly
               20                  25                  30
   Lys Pro Pro Gln Tyr Ile Ala Pro His Val Val Pro Asp Gln Leu Met
           35                  40                  45
   Pro Phe Gly Gly Ser Ser Glu Pro Cys Ala Leu Cys Ser Leu His Ser
       50                  55                  60
   Ile Gly Lys Ile Gly Gly Ala Gln Asn Arg Ser Tyr Ser Lys Leu Leu
   65                  70                  75                  80
   Cys Gly Leu Leu Ala Glu Arg Leu Arg Ile Ser Pro Asp Arg Val Tyr
                   85                  90                  95
   Ile Asn Tyr Tyr Asp Met Asn Ala Ala Asn Val Gly Trp Asn Asn Ser
               100                 105                 110
   Thr Phe Ala
           115
<210> 5
   <211> 115
   <212> PRT
   <213> Homo sapiens
<400> 5
   Met Pro Met Phe Ile Val Asn Thr Asn Val Pro Arg Ala Ser Val Pro
    1               5                  10                  15
   Asp Gly Phe Leu Ser Glu Leu Thr Gln Gln Leu Ala Gln Ala Thr Gly
               20                  25                  30
   Lys Pro Pro Gln Tyr Ile Ala Val His Val Val Pro Asp Gln Leu Met
           35                  40                  45
   Ala Phe Gly Gly Ser Ser Glu Pro Cys Ala Leu Cys Ser Leu His Ser
       50                  55                  60
   Ile Gly Lys Ile Gly Gly Ala Gln Asn Arg Ser Tyr Ser Lys Leu Leu
   65                  70                  75                  80
   Cys Gly Leu Leu Ala Glu Arg Leu Arg Ile Ser Pro Asp Arg Val Tyr
                   85                  90                  95
   Pro Asn Tyr Tyr Asp Met Asn Ala Ala Asn Val Pro Trp Asn Asn Ser
               100                 105                 110
   Thr Phe Ala
           115
<210> 6
   <211> 115
   <212> PRT
   <213> Homo sapiens
<220>
   <221> PEPTIDE
   <222> (41)
   <223> Xaa= Ala, Gly oder Ser
<220>
   <221> PEPTIDE
   <222> (46)
   <223> Xaa=Ala, Gly oder Ser
<400> 6
   Met Pro Met Phe Ile Val Asn Thr Asn Val Pro Arg Ala Ser Val Pro
    1               5                  10                  15
   Asp Gly Phe Leu Ser Glu Leu Thr Gln Gln Leu Ala Gln Ala Thr Gly
               20                  25                  30
   Lys Pro Pro Gln Tyr Ile Ala Val Xaa Val Val Pro Asp Xaa Leu Met
           35                  40                  45
   Ala Phe Gly Gly Ser Ser Glu Pro Cys Ala Leu Cys Ser Leu His Ser
       50                  55                  60
   Ile Gly Lys Ile Gly Gly Ala Gln Asn Arg Ser Tyr Ser Lys Leu Leu
   65                  70                  75                  80
   Cys Gly Leu Leu Ala Glu Arg Leu Arg Ile Ser Pro Asp Arg Val Tyr
                   85                  90                  95
   Ile Asn Tyr Tyr Asp Met Asn Ala Ala Asn Val Gly Trp Asn Asn Ser
               100                 105                 110
   Thr Phe Ala
           115
<210> 7
   <211> 115
   <212> PRT
   <213> Homo sapiens
<220>
   <221> PEPTIDE
   <222> (49)
   <223> Xaa= N alpha-Ala
<400> 7
   Met Pro Met Phe Ile Val Asn Thr Asn Val Pro Arg Ala Ser Val Pro
    1               5                  10                  15
   Asp Gly Phe Leu Ser Glu Leu Thr Gln Gln Leu Ala Gln Ala Thr Gly
                   20                  25                  30
   Lys Pro Pro Gln Tyr Ile Ala Val His Val Val Pro Asp Gln Leu Met
           35                  40                  45
   Xaa Phe Gly Gly Ser Ser Glu Pro Cys Ala Leu Cys Ser Leu His Ser
       50                  55                  60
   Ile Gly Lys Ile Gly Gly Ala Gln Asn Arg Ser Tyr Ser Lys Leu Leu
   65                  70                  75                  80
   Cys Gly Leu Leu Ala Glu Arg Leu Arg Ile Ser Pro Asp Arg Val Tyr
                   85                  90                  95
   Ile Asn Tyr Tyr Asp Met Asn Ala Ala Asn Val Gly Trp Asn Asn Ser
               100                 105                 110
   Thr Phe Ala
           115
<210> 8
   <211> 115
   <212> PRT
   <213> Homo sapiens
<220>
   <221> PEPTIDE
   <222> (108)
   <223> Xaa=N alpha-Gly
<220>
   <221> PEPTIDE
   <222> (49)
   <223> Xaa= N alpha -Ala
<400> 8
   Met Pro Met Phe Ile Val Asn Thr Asn Val Pro Arg Ala Ser Val Pro
   1 5 10 15
   Asp Gly Phe Leu Ser Glu Leu Thr Gln Gln Leu Ala Gln Ala Thr Gly
   20 25 30
   Lys Pro Pro Gln Tyr Ile Ala Val His Val Val Pro Asp Gln Leu Met
   35 40 45
   Xaa Phe Gly Gly Ser Ser Glu Pro Cys Ala Leu Cys Ser Leu His Ser
   50 55 60
   Ile Gly Lys Ile Gly Gly Ala Gln Asn Arg Ser Tyr Ser Lys Leu Leu
   65 70 75 80
   Cys Gly Leu Leu Ala Glu Arg Leu Arg Ile Ser Pro Asp Arg Val Tyr
   85 90 95
   Ile Asn Tyr Tyr Asp Met Asn Ala Ala Asn Val Xaa Trp Asn Asn Ser
   100 105 110
   Thr Phe Ala
   115
<210> 9
   <211> 115
   <212> PRT
   <213> Homo sapiens
<220>
   <221> PEPTIDE
   <222> (108)
   <223> Xaa= N alpha-Gly
<400> 9
   Met Pro Met Phe Ile Val Asn Thr Asn Val Pro Arg Ala Ser Val Pro
    1               5                  10                  15
   Asp Gly Phe Leu Ser Glu Leu Thr Gln Gln Leu Ala Gln Ala Thr Gly
               20                  25                  30
   Lys Pro Pro Gln Tyr Ile Ala Val His Val Val Pro Asp Gln Leu Met
           35                  40                  45
   Ala Phe Gly Gly Ser Ser Glu Pro Cys Ala Leu Cys Ser Leu His Ser
       50                  55                  60
   Ile Gly Lys Ile Gly Gly Ala Gln Asn Arg Ser Tyr Ser Lys Leu Leu
   65                  70                  75                      80
   Cys Gly Leu Leu Ala Glu Arg Leu Arg Ile Ser Pro Asp Arg Val Tyr
                   85                  90                  95
   Ile Asn Tyr Tyr Asp Met Asn Ala Ala Asn Val Xaa Trp Asn Asn Ser
               100                 105                 110
   Thr Phe Ala
           115
<210> 10
   <211> 115
   <212> PRT
   <213> Homo sapiens
<220>
   <221> PEPTIDE
   <222> (40)
   <223> Xaa=N alpha-Val
<220>
   <221> PEPTIDE
   <222> (49)
   <223> Xaa=N alpha-Ala
<400> 10
   Met Pro Met Phe Ile Val Asn Thr Asn Val Pro Arg Ala Ser Val Pro
   1 5 10 15
   Asp Gly Phe Leu Ser Glu Leu Thr Gln Gln Leu Ala Gln Ala Thr Gly
   20 25 30
   Lys Pro Pro Gln Tyr Ile Ala Xaa His Val Val Pro Asp Gln Leu Met
   35 40 45
   Xaa Phe Gly Gly Ser Ser Glu Pro Cys Ala Leu Cys Ser Leu His Ser
   50 55 60
   Ile Gly Lys Ile Gly Gly Ala Gln Asn Arg Ser Tyr Ser Lys Leu Leu
   65 70 75 80
   Cys Gly Leu Leu Ala Glu Arg Leu Arg Ile Ser Pro Asp Arg Val Tyr
   85 90 95
   Ile Asn Tyr Tyr Asp Met Asn Ala Ala Asn Val Gly Trp Asn Asn Ser
   100 105 110
   Thr Phe Ala
   115
<210> 11
   <211> 115
   <212> PRT
   <213> Homo sapiens
<220>
   <221> PEPTIDE
   <222> (97)
   <223> Xaa=N alpha-Ile
<220>
   <221> PEPTIDE
   <222> (108)
   <223> Xaa-N alpha-Gly
<400> 11
   Met Pro Met Phe Ile Val Asn Thr Asn Val Pro Arg Ala Ser Val Pro
   1 5 10 15
   Asp Gly Phe Leu Ser Glu Leu Thr Gln Gln Leu Ala Gln Ala Thr Gly
   20 25 30
   Lys Pro Pro Gln Tyr Ile Ala Val His Val Val Pro Asp Gln Leu Met
   35 40 45
   Ala Phe Gly Gly Ser Ser Glu Pro Cys Ala Leu Cys Ser Leu His Ser
   50 55 60
   Ile Gly Lys Ile Gly Gly Ala Gln Asn Arg Ser Tyr Ser Lys Leu Leu
   65 70 75 80
   Cys Gly Leu Leu Ala Glu Arg Leu Arg Ile Ser Pro Asp Arg Val Tyr
   85 90 95
   Xaa Asn Tyr Tyr Asp Met Asn Ala Ala Asn Val Xaa Trp Asn Asn Ser
   100 105 110
   Thr Phe Ala
   115
<210> 12
   <211> 115
   <212> PRT
   <213> Homo sapiens
<220>
   <221> PEPTIDE
   <222> (57)
   <223> Xaa= eine unnatürliche Aminosäure mit struktureller Seitenkettenähnlichkeit zu Vinylpyridin oder DTNB
<220>
   <221> PEPTIDE
   <222> (60)
   <223> Xaa= eine unnatürliche Aminosäure mit struktureller Seitenkettenähnlichkeit zu Vinylpyridin oder DTNB
<220>
   <221> PEPTIDE
   <222> (81)
   <223> Xaa=eine unnatürliche Aminosäure mit struktureller Seitenkettenähnlichkeit zu Vinylpyridin oder DTNB
<400> 12
   Met Pro Met Phe Ile Val Asn Thr Asn Val Pro Arg Ala Ser Val Pro
   1 5 10 15
   Asp Gly Phe Leu Ser Glu Leu Thr Gln Gln Leu Ala Gln Ala Thr Gly
   20 25 30
   Lys Pro Pro Gln Tyr Ile Ala Val His Val Val Pro Asp Gln Leu Met
   35 40 45
   Ala Phe Gly Gly Ser Ser Glu Pro Xaa Ala Leu Xaa Ser Leu His Ser
   50 55 60
   Ile Gly Lys Ile Gly Gly Ala Gln Asn Arg Ser Tyr Ser Lys Leu Leu
   65 70 75 80
   Xaa Gly Leu Leu Ala Glu Arg Leu Arg Ile Ser Pro Asp Arg Val Tyr
   85 90 95
   Ile Asn Tyr Tyr Asp Met Asn Ala Ala Asn Val Gly Trp Asn Asn Ser
   100 105 110
   Thr Phe Ala
   115
<210> 13
   <211> 115
   <212> PRT
   <213> Homo sapiens
<220>
   <221> PEPTIDE
   <222> (56)
   <223> Xaa= Lys, Orn, Dab, Dap oder Glu
<220>
   <221> PEPTIDE
   <222> (61)
   <223> Xaa=Asp, Lys, Orn, Dab, Dap oder Glu
<400> 13
   Met Pro Met Phe Ile Val Asn Thr Asn Val Pro Arg Ala Ser Val Pro
   1               5                   10                  15
   Asp Gly Phe Leu Ser Glu Leu Thr Gln Gln Leu Ala Gln Ala Thr Gly
               20                  25                  30
   Lys Pro Pro Gln Tyr Ile Ala Val His Val Val Pro Asp Gln Leu Met
           35                  40                  45
   Ala Phe Gly Gly Ser Ser Glu Xaa Cys Ala Leu Cys Xaa Leu His Ser
       50                  55                  60
   Ile Gly Lys Ile Gly Gly Ala Gln Asn Arg Ser Tyr Ser Lys Leu Leu
   65                  70                  75                  80
   Cys Gly Leu Leu Ala Glu Arg Leu Arg Ile Ser Pro Asp Arg Val Tyr
                   85                  90                  95
   Ile Asn Tyr Tyr Asp Met Asn Ala Ala Asn Val Gly Trp Asn Asn Ser
               100                 105                 110
   Thr Phe Ala
           115
<210> 14
   <211> 115
   <212> PRT
   <213> Homo sapiens
<220>
   <221> PEPTIDE
   <222> (55)
   <223> Xaa=Lys, Asp, Örn, Dab, Dap oder Glu
<220>
   <221> PEPTIDE
   <222> (62)
   <223> Xaa= Asp, Lys, Orn, Dab, Dap oder Glu
<400> 14
   Met Pro Met Phe Ile Val Asn Thr Asn Val Pro Arg Ala Ser Val Pro
    1               5                  10                  15
   Asp Gly Phe Leu Ser Glu Leu Thr Gln Gln Leu Ala Gln Ala Thr Gly
               20                  25                  30
   Lys Pro Pro Gln Tyr Ile Ala Val His Val Val Pro Asp Gln Leu Met
           35                  40                  45
   Ala Phe Gly Gly Ser Ser Xaa Pro Cys Ala Leu Cys Ser Xaa His Ser
       50                  55                  60
   Ile Gly Lys Ile Gly Gly Ala Gln Asn Arg Ser Tyr Ser Lys Leu Leu
   65                  70                  75                  80
   Cys Gly Leu Leu Ala Glu Arg Leu Arg Ile Ser Pro Asp Arg Val Tyr
                   85                  90                  95
   Ile Asn Tyr Tyr Asp Met Asn Ala Ala Asn Val Gly Trp Asn Asn Ser
               100                 105                 110
   Thr Phe Ala
           115
<210> 15
   <211> 115
   <212> PRT
   <213> Homo sapiens
<220>
   <221> PEPTIDE
   <222> (56)
   <223> Xaa=Lys, Asp, Orn, Dab. Dap oder Glu
<220>
   <221> PEPTIDE
   <222> (57)
   <223> Xaa=Ser, Ala oder Gly
<220>
   <221> PEPTIDE
   <222> (60)
   <223> Xaa=Ser, Ala oder Gly
<220>
   <221> PEPTIDE
   <222> (61)
   <223> Xaa=Asp, Lys, Orn, Dab, Dap oder Glu
<400> 15
   Met Pro Met Phe Ile Val Asn Thr Asn Val Pro Arg Ala Ser Val Pro
   1 5 10 15
   Asp Gly Phe Leu Ser Glu Leu Thr Gln Gln Leu Ala Gln Ala Thr Gly
   20 25 30
   Lys Pro Pro Gln Tyr Ile Ala Val His Val Val Pro Asp Gln Leu Met
   35 40 45
   Ala Phe Gly Gly Ser Ser Glu Xaa Xaa Ala Leu Xaa Xaa Leu His Ser
   50 55 60
   Ile Gly Lys Ile Gly Gly Ala Gln Asn Arg Ser Tyr Ser Lys Leu Leu
   65 70 75 80
   Cys Gly Leu Leu Ala Glu Arg Leu Arg Ile Ser Pro Asp Arg Val Tyr
   85 90 95
   Ile Asn Tyr Tyr Asp Met Asn Ala Ala Asn Val Gly Trp Asn Asn Ser
   100 105 110
   Thr Phe Ala
   115
<210> 16
   <211> 115
   <212> PRT
   <213> Homo sapiens
<220>
   <221> PEPTIDE
   <222> (55)
   <223> Xaa=Lys, Asp, Orn, Dab, Dap oder Glu
<220>
   <221> PEPTIDE
   <222> (57)
   <223> Xaa=Ser, Ala oder Gly
<220>
   <221> PEPTIDE
   <222> (60)
   <223> Xaa=Ser, Ala oder Gly
<220>
   <221> PEPTIDE
   <222> (62)
   <223> Xaa=Asp, Lys, Orn, Dab, Dap oder Glu
<400> 16
   Met Pro Met Phe Ile Val Asn Thr Asn Val Pro Arg Ala Ser Val Pro
   1 5 10 15
   Asp Gly Phe Leu Ser Glu Leu Thr Gln Gln Leu Ala Gln Ala Thr Gly
   20 25 30
   Lys Pro Pro Gln Tyr Ile Ala Val His Val Val Pro Asp Gln Leu Met
   35 40 45
   Ala Phe Gly Gly Ser Ser Xaa Pro Xaa Ala Leu Xaa Ser Xaa His Ser
   50 55 60
   Ile Gly Lys Ile Gly Gly Ala Gln Asn Arg Ser Tyr Ser Lys Leu Leu
   65 70 75 80
   Cys Gly Leu Leu Ala Glu Arg Leu Arg Ile Ser Pro Asp Arg Val Tyr
   85 90 95
   Ile Asn Tyr Tyr Asp Met Asn Ala Ala Asn Val Gly Trp Asn Asn Ser
   100 105 110
   Thr Phe Ala
   115
<210> 17
   <211> 16
   <212> PRT
   <213> Homo sapiens
<400> 17
   Phe Gly Gly Ser Ser Glu Pro Cys Ala Leu Cys Ser Leu His Ser Ile
   1 5 10 15
<210> 18
   <211> 16
   <212> PRT
   <213> Homo sapiens
<400> 18
   Gly Gly Ser Ser Glu Pro Cys Ala Leu Cys Ser Leu His Ser Ile Gly
   1 5 10 15
<210> 19
   <211> 11
   <212> PRT
   <213> Homo sapiens
<400> 19
   Glu Pro Cys Ala Leu Cys Ser Leu His Ser Ile
   1 5   10
<210> 20
   <211> 12
   <212> PRT
   <213> Homo sapiens
<400> 20
   Ser Glu Pro Cys Ala Leu Cys Ser Leu His Ser Ile
   1 5 10
<210> 21
   <211> 13
   <212> PRT
   <213> Homo sapiens
<400> 21
   Ser Ser Glu Pro Cys Ala Leu Cys Ser Leu His Ser Ile
   1 5 10
<210> 22
   <211> 14
   <212> PRT
   <213> Homo sapiens
<400> 22
   Gly Ser Ser Glu Pro Cys Ala Leu Cys Ser Leu His Ser Ile
   1 5 10
<210> 23
   <211> 15
   <212> PRT
   <213> Homo sapiens
<400> 23
   Gly Gly Ser Ser Glu Pro Cys Ala Leu Cys Ser Leu His Ser Ile
   1 5 10 15
<210> 24
   <211> 16
   <212> PRT
   <213> Homo sapiens
<220>
   <221> PEPTIDE
   <222> (8)
   <223> Xaa= Ser, Ala oder Gly
<220>
   <221> PEPTIDE
   <222> (11)
   <223> Xaa= Ser, Ala oder Gly
<400> 24
   Phe Gly Gly Ser Ser Glu Pro Xaa Ala Leu Xaa Ser Leu His Ser Ile
    1               5                  10                  15
<210> 25
   <211> 115
   <212> PRT
   <213> Homo sapiens
<400> 25
   Met Pro Met Phe Ile Val Asn Thr Asn Val Pro Arg Ala Ser Val Pro
    1               5                  10                  15
   Asp Gly Phe Leu Ser Glu Leu Thr Gln Gln Leu Ala Gln Ala Thr Gly
                20                  25                  30
   Lys Pro Pro Gln Tyr Ile Ala Val His Val Val Pro Asp Gln Leu Met
            35                  40                  45
   Ala Phe Gly Gly Ser Ser Glu Pro Ser Ala Leu Ser Ser Leu His Ser
        50                  55                  60
   Ile Gly Lys Ile Gly Gly Ala Gln Asn Arg Ser Tyr Ser Lys Leu Leu
   65                  70                  75                  80
   Ser Gly Leu Leu Ala Glu Arg Leu Arg Ile Ser Pro Asp Arg Val Tyr
                   85                  90                  95
   Ile Asn Tyr Tyr Asp Met Asn Ala Ala Asn Val Gly Trp Asn Asn Ser
               100                 105                 110
   Thr Phe Ala
           115
<210> 26
   <211> 115
   <212> PRT
   <213> Homo sapiens
<400> 26
   Met Pro Met Phe Ile Val Asn Thr Asn Val Pro Arg Ala Ser Val Pro
   1 5 10 15
   Asp Gly Phe Leu Ser Glu Leu Thr Gln Gln Leu Ala Gln Ala Thr Gly
   20 25 30
   Lys Pro Pro Gln Tyr Ile Ala Val His Val Val Pro Asp Gln Leu Met
   35 40 45
   Ala Phe Gly Gly Ser Ser Glu Pro Ala Ala Leu Ala Ser Leu His Ser
   50 55 60
   Ile Gly Lys Ile Gly Gly Ala Gln Asn Arg Ser Tyr Ser Lys Leu Leu
   65 70 75 80
   Ala Gly Leu Leu Ala Glu Arg Leu Arg Ile Ser Pro Asp Arg Val Tyr
   85 90 95
   Ile Asn Tyr Tyr Asp Met Asn Ala Ala Asn Val Gly Trp Asn Asn Ser
   100 105 110
   Thr Phe Ala
   115
<210> 27
   <211> 115
   <212> PRT
   <213> Homo sapiens
<400> 27
   Met Pro Met Phe Ile Val Asn Thr Asn Val Pro Arg Ala Ser Val Pro
   1 5 10 15
   Asp Gly Phe Leu Ser Glu Leu Thr Gln Gln Leu Ala Gln Ala Thr Gly
   20 25 30
   Lys Pro Pro Gln Tyr Ile Ala Val His Val Val Pro Asp Gln Leu Met
   35 40 45
   Ala Phe Gly Gly Ser Ser Glu Pro Phe Ala Leu Phe Ser Leu His Ser
   50 55 60
   Ile Gly Lys Ile Gly Gly Ala Gln Asn Arg Ser Tyr Ser Lys Leu Leu
   65 70 75 80
   Phe Gly Leu Leu Ala Glu Arg Leu Arg Ile Ser Pro Asp Arg Val Tyr
   85 90 95
   Ile Asn Tyr Tyr Asp Met Asn Ala Ala Asn Val Gly Trp Asn Asn Ser
   100 105 110
   Thr Phe Ala
   115
<210> 28
   <211> .115
   <212> PRT
   <213> Homo sapiens
<400> 28
<210> 29
   <211> 115
   <212> PRT
   <213> Homo sapiens
<400> 29
   Met Pro Met Phe Ile Val Asn Thr Asn Val Pro Arg Ala Ser Val Pro
   1 5 10 15
   Asp Gly Phe Leu Ser Glu Leu Thr Gln Gln Leu Ala Gln Ala Thr Gly
   20 25 30
   Lys Pro Pro Gln Tyr Ile Ala Val His Val Val Pro Asp Gln Leu Met
   35 40 45
   Ala Phe Gly Gly Ser Ser Glu Pro Tyr Ala Leu Tyr Ser Leu His Ser
   50 55 60
   Ile Gly Lys Ile Gly Gly Ala Gln Asn Arg Ser Tyr Ser Lys Leu Leu
   65 70 75 80
   Tyr Gly Leu Leu Ala Glu Arg Leu Arg Ile Ser Pro Asp Arg Val Tyr
   85 90 95
   Ile Asn Tyr Tyr Asp Met Asn Ala Ala Asn Val Gly Trp Asn Asn Ser
   100 105 110
   Thr Phe Ala
   115
<210> 30
   <211> 115
   <212> PRT
   <213> Homo sapiens
<220>
   <221> PEPTIDE
   <222> (57)
   <223> Xaa= Cys-Rest, der mit einem Alkylierungsrest modifiziert wurde
<220>
   <221> PEPTIDE
   <222> (60)
   <223> Xaa= Cys-Rest, der mit einem Alkylierungsrest modifiziert wurde
<220>
   <221> PEPTIDE
   <222> (81)
   <223> Xaa= Cys-Rest, der mit einem Alkylierungsrest modifiziert wurde
<400> 30
   Met Pro Met Phe Ile Val Asn Thr Asn Val Pro Arg Ala Ser Val Pro
   1 5 10 15
   Asp Gly Phe Leu Ser Glu Leu Thr Gln Gln Leu Ala Gln Ala Thr Gly
   20 25 30
   Lys Pro Pro Gln Tyr Ile Ala Val His Val Val Pro Asp Gln Leu Met
   35 40 45
   Ala Phe Gly Gly Ser Ser Glu Pro Xaa Ala Leu Xaa Ser Leu His Ser
   50 55 60
   Ile Gly Lys Ile Gly Gly Ala Gln Asn Arg Ser Tyr Ser Lys Leu Leu
   65 70 75 80
   Xaa Gly Leu Leu Ala Glu Arg Leu Arg Ile Ser Pro Asp Arg Val Tyr
   85 90 95
   Ile Asn Tyr Tyr Asp Met Asn Ala Ala Asn Val Gly Trp Asn Asn Ser
   100 105 110
   Thr Phe Ala
   115
<210> 31
   <211> 21
   <212> PRT
   <213> Homo sapiens
<220>
   <221> PEPTIDE
   <222> (9) .. (12)
   <223> Xaa steht für kleine Aminosäuren wie Gly oder Aminosäuren, die häufig in beta-Turns anzutreffen sind, oder Aminosäuren, die zur Lactambrückenbildung genutzt werden können, wie Asp, Glu, Lys, Orn us
<400> 31
   Met Pro Met Phe Ile Val Asn Thr Xaa Xaa Xaa Xaa Cys Ala Leu Cys
   1 5 10 15
   Ser Leu His Ser Ile
   20
<210> 32
   <211> 22
   <212> PRT
   <213> Homo sapiens
<220>
   <221> PEPTIDE
   <222> (9)..(13)
   <223> Xaa steht für kleine Aminosäuren wie Gly oder Aminosäuren, die häufig in beta-Turns anzutreffen sind, oder Aminosäuren, die zur Lactambrückenbildung genutzt werden können, wie Asp, Glu, Lys, Orn us
<400> 32
   Met Pro Met Phe Ile Val Asn Thr Xaa Xaa Xaa Xaa Xaa Cys Ala Leu
   1 5 10 15
   Cys Ser Leu His Ser Ile
   20
<210> 33
   <211> 23
   <212> PRT
   <213> Homo sapiens
<220>
   <221> PEPTIDE
   <222> (9)..(14)
   <223> Xaa steht für kleine Aminosäuren wie Gly oder Aminosäuren, die häufig in beta-Turns anzutreffen sind, oder Aminosäuren, die zur Lactambrückenbildung genutzt werden können, wie Asp, Glu, Lys, Orn usw
<400> 33
   Met Pro Met Phe Ile Val Asn Thr Xaa Xaa Xaa Xaa Xaa Xaa Cys Ala
   1 5 10 15
   Leu Cys Ser Leu His Ser Ile
   20
<210> 34
   <211> 22
   <212> PRT
   <213> Homo sapiens
<220>
   <221> PEPTIDE
   <222> (10)..(12)
   <223> Xaa steht für kleine Aminosäuren wie Gly oder Aminosäuren, die häufig in beta-Turns anzutreffen sind, oder Aminosäuren, die zur Lactambrückenbildung genutzt werden können, wie Asp, Glu, Lys, Orn usw.
<400> 34
   Met Pro Met Phe Ile Val Asn Thr Asn Xaa Xaa Xaa Pro Cys Ala Leu
   1 5 10 15
   Cys Ser Leu His Ser Ile
   20
<210> 35
   <211> 23
   <212> PRT
   <213> Homo sapiens
<220>
   <221> PEPTIDE
   <222> (10)..(13)
   <223> Xaa steht für kleine Aminosäuren wie Gly oder Aminosäuren, die häufig in beta-Turns anzutreffen sind, oder Aminosäuren, die zur Lactambrückenbildung genutzt werden können, wie Asp, Glu, Lys, Orn usw.
<400> 35
   Met Pro Met Phe Ile Val Asn Thr Asn Xaa Xaa Xaa Xaa Pro Cys Ala
    1               5                  10                  15
   Leu Cys Ser Leu His Ser Ile
               20
<210> 36
   <211> 24
   <212> PRT
   <213> Homo sapiens
<220>
   <221> PEPTIDE
   <222> (10)..(14)
   <223> Xaa steht für kleine Aminosäuren wie Gly oder Aminosäuren, die häufig in beta-Turns anzutreffen sind, oder Aminosäuren, die zur Lactambrückenbildung genutzt werden können, wie Asp, Glu, Lys, Orn usw.
<400> 36
   Met Pro Met Phe Ile Val Asn Thr Asn Xaa Xaa Xaa Xaa Xaa Pro Cys
    1               5                  10                  15
   Ala Leu Cys Ser Leu His Ser Ile
               20
<210> 37
   <211> 25
   <212> PRT
   <213> Homo sapiens
<220>
   <221> PEPTIDE
   <222> (10)..(15)
   <223> Xaa steht für kleine Aminosäuren wie Gly oder Aminosäuren, die häufig in beta-Turns anzutreffen sind, oder Aminosäuren, die zur Lactambrückenbildung genutzt werden können, wie Asp, Glu, Lys, Orn usw.
<400> 37
   Met Pro Met Phe Ile Val Asn Thr Asn Xaa Xaa Xaa Xaa Xaa Xaa Pro
    1               5                  10                  15
   Cys Ala Leu Cys Ser Leu His Ser Ile
               20                  25

## Beschreibung

Die vorliegende Erfindung betrifft Analoga, Agonisten, Antagonisten, Inhibitoren, Modulatoren und Varianten sowie Teilsequenzen des eine Oxidoreduktase-Enzymaktivität aufweisenden Makrophagen-Migra tions-Inhibitions-Faktors (MIF-analoge Redoxstoffe MIFARs) sowie die Verwendung dieser in Diagnostik-, Therapie- und Screeningverfahren.

Das angeborene und das adaptive Immunsystem stehen im engen Wechselverhältnis. Von Lymphocyten gebildete Antikörper - Teil des adaptiven Immunsystems - unterstützen die Phagocyten - Teil des angeborenen Immunsystems -, ihre Ziele zu erkennen. Nach einer klonalen Aktivierung durch Antigene produzieren T-Lymphocyten Lymphokine, welche ihrerseits Phagocyten dazu stimulieren, Infektionserreger sehr effektiv zu zerstören. Macrophagen wiederum leisten den Lymphocyten Hilfestellung, indem sie Antigene von der Peripherie zu den Lymphknoten und anderen lymphatischen Organen transportieren, wo sie den Lymphocyten so dargeboten werden, dass sie diese identifizieren. Die Lymphocyten sind in der Lage, Polypeptide freizusetzen - darunter den sogenannten Magrophagen-Migrations-Inhibitions-Faktor (MIF).

Historisch stellte man fest, dass MIF die weitere Migration der Macrophagen verhindert, wenn diese die Infektionsstelle erreicht haben. Die Folge sind Entzündungs- und Immunabwehrreaktionen direkt an der Infektionsstelle.

Mitte der 60er Jahre wurde MIF vor allem phänomenologisch als "biologische Aktivität mit Macrophagen migrationsinhibierenden Eigenschaften" untersucht (David, 1966; Bloom and Bennett, 1966). Eine molekulare Identifizierung von MIF begann erst mit der Klonierung einer T-Zell-MIF cDNA im Jahre 1989 (Weiser, et al., 1989). Durch eine Reihe von Entdeckungen der letzten Jahre ist MIF heute nicht nur als T-Zell-stämmiger Inhibitor der Macrophagen-Migration bekannt, sondern gilt als wichtiger proinflammatorischer Immunfaktor mit breitem Bildungs- und Wirkungsspektrum (Bernhagen et al., 1998). Insbesondere zeichnet sich MIF durch seine antagonisierende Wirkung der antiinflammatorischen und immunsuppressiven Effekte der Glucocorticoide aus (Calandra et al., 1995, Bacher et al., 1996). MIF gilt als Mediator beziehungsweise Co-Mediator verschiedener Krankheitszustände, wie dem septischen Schock (Bernhagen et al., 1993), der Spättyp-Hypersensitivität (DTH) (Bernhagen et al., 1996), des Adult Respiratory Distress Syndrome (Donnelly et al., 1997), der Glomerulonephritis (Lan et al., 1997), der rheumatoiden Arthritis (Mikulowska et al., 1997; Leech et al., 1998) und anderen pathologischen Veränderungen und Krankheiten.

Die Beteiligung von MIF bei der Genese von Krankheiten ist für den bakteriellen septischen Schock am besten abgesichert. Zahlreiche Veröffentlichungen belegen, dass rekombinantes MIF die bakterielle oder Endotoxin-Sepsis in Mäusen und Ratten stark erhöht und dass poly- und monoklonale Anti-MIF-Antikörper die Tiere vor Sepsis signifikant schützen, sowie dass MIF-Knockout-Mäuse gegen bakterielle Sepsis geschützt sind (Calandra et al., 2000; Bernhagen et al., 1993; Bernhagen et al., 1994; Bozza et al., 1999).

MIF besteht aus 114 Aminosäuren und läßt sich keiner bekannten Cytokinfamilie zuordnen, wodurch überlappende Rezeptoraktivitäten ausgeschlossen sind. Darüber hinaus ist MIF durch seine enzymatische und Glucocorticoid-antagonisierende Wirkungsweise einzigartig unter den Cytokinen.

Der molekulare Wirkmechanismus von MIF ist noch nicht im Detail geklärt. Insbesondere wurde bis zum gegenwärtigen Zeitpunkt noch kein Membranrezeptor (Cytokinrezeptor) zur Vermittlung der MIF-Wirkungen an Zielzellen identifiziert. Die aktuelle Entdeckung der katalytischen Fähigkeiten von MIF deutet darauf hin, dass die Immun- und proinflammatorischen Wirkungen von MIF in-vitro außer durch rezeptorvermittelte Mechanismen auch enzymatisch oder ausschließlich enzymatisch vermittelt werden (Bernhagen et al., 1998; Kleemann et al., 1998a; Kleemann et al., 1998b; Kleemann et al., 1999). Neben dieser Thiolprotein-Oxidoreduktase-Enzymaktivität wurde noch eine weitere Enzymfunktion, eine Tautomeraseaktivität, für MIF gefunden (Rosengren et al., 1996; Rosengren et al., 1997; Swope et al., 1998). Der Zusammenhang zwischen diesen beiden Enzymaktivitäten ist noch nicht aufgeklärt.

Die Tautomeraseaktivität wird unter anderem in der-WO 97/29635 beschrieben. Die immunologische und physiologische Relevanz der Tautomeraseaktivität ist in der Literatur umstritten (Swope et al., 1998; Bendrat et al., 1997; Hermanowski-Vosatka et al., 1999). Die WO 94/26307, AU 692159, WO 98/17314, JP 09077799, US 6,080,407 und die WO 96/15242 offenbaren therapeutische und diagnostische Anwendungen von MIF, die zum Teil auf der Tautomeraseaktivität basieren, aber vor allem auf der Verwendung von Anti-MIF-Antikörpern sowie löslichen MIF-Rezeptoren beziehungsweise MIF-Antisense RNA-Molekülen; diesen Offenbarungen liegt die Annahme zugrunde, dass MIF-Wirkungen über klassische rezeptorvermittelte Wege verlaufen. Therapeutische Ansätze auf der Basis von Antikörpern haben jedoch bekanntermaßen eine Vielzahl von Nachteilen, die unter anderem auf die molekulare Größe und somit (i) umständliche Applizierbarkeit, (ii) die schnelle Metabolisierung und (iii) mögliche Immunreaktionen des Patienten gegen den therapeutischen Antikörper selbst zurückgehen.

Die US 5, 656, 737, WO 97/18229, WO 96/09389, WO 95/3100468 und die US 7, 691, 191 offenbaren verschiedene Anwendungsmöglichkeiten für MIF; hierbei handelt es sich einerseits um sehr spezialisierte Verfahren, zum Beispiel zur Herstellung von rekombinantem MIF und andererseits um Offenbarungen mit eingeschränkten Anwendungsbereichen, zum Beispiel zur Bekämpfung von Parasiten oder zur Erzeugung eines MIFs aus Augenlinsen.

Ansätze auf der Basis von Nucleinsäuren, wie zum Beispiel Antisense-RNA, haben den Nachteil, dass RNA inhärent instabil ist und durch komplizierte chemische Methoden erst stabilisiert werden muß, bevor eine effiziente Applikation erfolgen kann. Der therapeutische Erfolg bei Antisense-RNA-Verfahren ist zudem bisher wenig belegt.

In Anlehnung an Ansätze bei anderen Cytokinen wäre in der Krankheitsdiagnostik noch eine weitere MIF-basierte Strategie denkbar. Dies ist die Erstellung von MIF-mRNA-Expressionsprofilen unter Einsatz von MIF-basierten Sonden. Da MIF-mRNA jedoch in den meisten Zellen in sehr hohen Mengen konstitutiv exprimiert wird, sind die Empfindlichkeit und die Aussagekraft solcher Profile beziehungsweise die zu erwartenden geringen Veränderungen als nachteilig einzuschätzen.

Keine der Druckschriften offenbart konkrete MIF-basierte Anwendungsmöglichkeiten auf der Basis der Oxidoreduktaseaktivität von MIF. Therapeutische Anwendungen dieser Aktivität von MIF gibt es bisher nicht. In der klinischen Diagnostik werden MIF-basierte Methoden ebenfalls nicht eingesetzt.

Mögliche therapeutische Anwendungen von MIF selbst sind vor allem deshalb problematisch, weil sich Cytokine durch eine pleiotrope Wirkungsweise auszeichnen. Außerdem gibt es innerhalb der Wirkungen der Cytokine ein hohes Maß an Redundanz. Das bedeutet, dass mehrere Cytokine zum Teil weitreichende überlappende Effekte aufweisen. Das Ausschalten einzelner Cytokine, zum Beispiel durch Antagonisten oder ähnliches, ist daher therapeutisch oft nicht sehr wirkungsvoll. Aus demselben Grund korrelieren Cytokinanalysen in der Diagnostik oft nicht eindeutig mit bestimmten Krankheiten.

MIF selbst kommt daher aufgrund seiner breiten proinflammatorischen Wirkung für die meisten Krankheiten nicht als therapeutisches Protein in Frage. Ausnahmen wären krankhafte immunsuppressive Situationen sowie mit Glucocorticoiden behandelte Krankheiten, zum Beispiel chronische Erkrankungen, bei welchen MIF endo- oder exogen-induzierte Glucocorticoid-vermittelte Immunsuppressionen antagonisieren könnte. Die Applikation von rekombinantem humanem MIF bringt jedoch die bekannten Nachteile einer Applikation eines Proteins solcher Größe mit sich. Hier sind vor allem (i) die schnelle Metabolisierung, (ii) die Abbaurate oder mögliche (iii) Abwehrreaktionen zu nennen.

Die Anwendung von MIF in diagnostischen Assays in der Klinik ist zwar denkbar, doch weisen die Empfindlichkeitsgrenzen (> 1 ng/ml) darauf hin, dass der bisher entwickelte ELISA-Ansatz für klinische Anwendungen nicht ausreichend empfindlich ist. Der ELISA ist darüber hinaus sehr anfällig für Störungen, insbesondere ELISA-Assays aus Blut beziehungsweise Serum sind nicht verläßlich genug.

Bezüglich der Oxidoreduktaseaktivität von MIF könnten Daten aus der Literatur, die gezeigt haben, dass die gentechnisch hergestellte MIF-Mutante C60S in einem Immunassay nur teilweise aktiv war, darauf hinweisen, dass diese Mutante therapeutisch als MIF-Antagonist eingesetzt werden könnte (Kleemann et al., 1998a, 1999). MIF C60S weist in ihrem Oxidoreduktase-Zentrum einen Austausch des Cys an Position 60 durch eine eine primäre Alkoholfunktion aufweisende natürliche Aminosäure, nämlich Serin, auf. Diese Mutante ist nur unter aufwendigsten und ungewöhnlichen Bedingungen löslich (siehe Kleemann et al. 1998, 1999). Sie ist daher für den therapeutischen Einsatz nicht geeignet. Die Verwendung dieser Mutante für die Diagnostik ist aus denselben Gründen nachteilig.

Zum gegenwärtigen Zeitpunkt sind deshalb MIF-basierte Strategien einer Therapie und Diagnose von menschlichen Krankheiten nicht verfügbar.

Demgemäß stehen bisher keine Substanzen zu Verfügung, die als Analoga, Agonisten, Antagonisten, Inhibitoren und Varianten des Oxidoreduktase-Enzymaktivität aufweisenden MIFs als Immunmodulatoren, Therapeutika, Diagnostika und Screening-Agenzien bei inflammatorischen und Immunerkrankungen zum Einsatz kommen können.

Das der vorliegenden Erfindung zugrundeliegende technische Problem besteht also darin, Agenzien auf der Basis von MIF bereitzustellen, die sich für eine therapeutische, diagnostische oder Screening-Anwendung eignen.

Die vorliegende Erfindung löst dieses technische Problem durch die Bereitstellung eines Derivats nach dem Hauptanspruch, insbesondere eines Derivats eines ein Oxidoreduktase-Zentrum mit den Aminosäuren N-Terminus... Cys-Ala-Leu-Cys ...C-Terminus an den Positionen 57, 58, 59 und 60 aufweisenden MIFs, wobei dieses in löslicher Form vorliegt, wobei die Aktivität des Oxidoreduktase-Zentrums des MIFs modifiziert ist und das lösliche Derivat aus 16 Aminosäuren besteht und im Oxidoreduktase-Zentrum, also in dem den Aminosäuren 57 bis 60 des Wildtyp-MIFs entsprechenden Bereich, keine natürliche Aminosäure, insbesondere überhaupt keine Aminosäure, mit einer primären Alkoholfunktion, insbesondere einer Alkoholfunktion, aufweist. Offenbart, aber nicht zur Erfindung gehörig sind Derivate, die aus mindestens 11 Aminosäuren bestehen.

Der als Oxidoreduktase-Zentrum bezeichnete Bereich der erfindungsgemäßen auch als MIFARs bezeichneten MIF-Derivate ist der Bereich der MIFARs, der dem Oxidoreduktase-Zentrum des Wildtyp-MIF an den Positionen 57 bis 60 entspricht, also bei einem Sequenzvergleich des gesamten MIFARs mit dem Wildtyp-MIF als der Bereich erkannt wird, der an der Position des Wildtyp-Oxidoreduktase-Bereichs 57-60 vorliegt. Dieser Sequenzvergleich beziehungsweise das Sequenzalignment wird so durchgeführt, dass sich unter Berücksichtigung konservativer Aminoaustausche größtmögliche übereinstimmung zwischen dem MIFAR und dem Wildtyp-MIF ergibt.

Die Erfindung stellt bisher nicht bekannte MIFARs bereit, die die Oxidoreduktaseaktivität von MIF antagonisieren, modulieren, hemmen oder mimikrieren, wobei dies Analoga, Antagonisten, Inhibitoren, Varianten, chemisch modifizierte Derivate sowie peptidische Teilsequenzen und chemisch modifizierte Analoga von MIF umfasst, welche die Oxidoreduktaseaktivität von MIF antagonisieren, modulieren, hemmen oder mimikrieren. Eingeschlossen sind weiterhin gentherapeutisch einsetzbare Nucleinsäuren, zum Beispiel Gene, DNAs, cDNAs oder Exons, die für bestimmte MIF-Redox-Analoga kodieren sowie auch MIF-ähnliche Cytokine mit Oxidoreduktase-Aktivität (Redoxkine) . Bei den erfindungsgemäßen Analoga, Antagonisten, Agonisten, Inhibitoren, Modulatoren, Varianten und Teilsequenzen handelt es sich also um Peptide, Polypeptide und Antikörper, modifizierte Peptide und Polypeptidanaloga beziehungsweise Proteinanaloga des Macrophagen Migrations-Inhibitions-Faktors (MIF). Diese Stoffe werden im folgenden als Derivat eines MIFs oder MIF-analoge Redoxstoffe beziehungsweise MIFARs bezeichnet, da sie Analoga beziehungsweise Inhibitoren bezüglich der Oxidoreduktase-Enzymaktivität von MIF sind. Eingeschlossen in den Gegenstand der vorliegenden Lehre sind auch MIF-ähnliche Cytokine mit Oxidoreduktase-Aktivität (Redoxkine) sowie gentherapeutisch einsetzbare Nucleinsäuren, zum Beispiel Gene, DNAs, cDNAs oder Exons, die für bestimmte MIF-Redox-Analoga oder Inhibitoren kodieren.

Wegen des einzigartigen Struktur-Wirkungs-Spektrums des MIFs als enzymatisch redoxaktives Cytokin mit breitem Wirkungsspektrum in der Immun- und Entzündungsantwort des Wirtes und insbesondere seiner Glucocorticoid-antagonisierenden Effekte sind die erfindungsgemäßen MIF-basierten Therapiestrategien zur Behandlung einer Vielzahl von Immun-, Entzündungs- oder Krebskrankheiten vorteilhaft - ebenso wie MIF-basierte Methoden für die Diagnose dieser Krankheiten. Darüber hinaus sind die erfindungsgemäßen MIFARs für Screening-Anwendungen zur Identifizierung von potentiellen Medikamenten für die vorgenannten Krankheiten geeignet. In Forschungs- und Entwicklungslaboren können MIF-basierte Diagnostikkits für die Aufklärung immunologischer und biologischer Mechanismen sehr wichtig sein, da MIF bei vielen der erwähnten Mechanismen eine entscheidende regulatorische Rolle spielt.

Aus der Kombination der natürlichen immunologischen Funktionen von MIF und den beobachteten ungewöhnlichen Redox-Enzymaktivitäten resultieren neue Therapie- und diagnostische Ansätze für Krankheiten, die bis zum gegenwärtigen Zeitpunkt bekannten Cytokinbasierten Therapie- und Diagnoseverfahren überlegen sein können. Hierzu zählt auch der Befund, dass monomeres MIF sehr wirksam ist.

Die Aktivität des Oxidoreduktase-Zentrums des MIFs wird erfindungsgemäß auf verschiedene Weise modifiziert. Diese Modifizierung kann die Veränderung der Primärstruktur genauso einschließen wie die Veränderungen höherer Strukturen und/oder der Konformation. Erfindungsgemäß kann die Aktivität des Oxidoreduktase-Zentrums des MIFs entweder durch Modifizierung der Aminosäuresequenz des Oxidoreduktase-zentrums oder durch Modifizierung der für die Trimerisierung des MIFs notwendigen Aminosäuren durchgeführt werden. Im Zusammenhang mit der vorliegenden Erfindung wird unter einer Modifikation entweder ein Aminosäureaustausch und/oder eine Alkylierung oder eine Nα-Methylierung verstanden.

Im Zusammenhang mit der vorliegenden Erfindung wird unter einem Oxidoreduktase-Zentrum der Bereich 57 bis 60 des humanen MIFs mit den Aminosäuren (C-Terminus) Cys Ala Leu Cys (N-Terminus) oder eine dieser .Aminosäuresequenz oder davon abgeleiteten Sequenz entsprechende Region eines Derivats von MIF verstanden, wobei das Oxidoreduktase-Zentrum selbstverständlich im erfindungsgemäßen Derivat eine andere Lokalisierung aufweisen kann.

Im Zusammenhang mit der vorliegenden Erfindung wird unter löslich verstanden, dass das Derivat in physiologischen Puffern wie PBS oder TBS sowie auch in H₂O löslich ist. Die Derivate sind auch bei weit höheren Konzentrationen (MIF 50-65 zum Beispiel bei 1 mM vs. ≤ 8 µm bei C60S MIF) löslich.

Im Zusammenhang mit der vorliegenden Erfindung werden unter natürlichen Aminosäuren die 20 Aminosäuren verstanden, die regelmäßig in natürlicherweise vorkommenden Proteinen gefunden werden, und zwar Glycin, L-Alanin, L-Valin, L-Leucin, L-Isoleucin, L-Phenylalanin, L-Prolin, L-Serin, L-Treonin, L-Cystein, L-Methionin, L-Tryptophan, L-Tyrosin, L-Asparagin, L-Glutamin, L-Asparaginsäure, L-Glutaminsäure, L-Lysin, L-Arginin und L-Histidin. Unnatürliche Aminosäuren im Zusammenhang mit der vorliegenden Erfindung sind alle Aminosäuren, die im Sinne der vorstehenden Definition nicht natürlich sind. Im Zusammenhang mit der vorliegenden Erfindung wird unter einer primären Alkoholfunktion eine Hydroxylgruppe an einem primären, also einem mit nur einem Kohlenstoffatom verbundenen, Kohlenstoffatom verstanden, wobei dieses Kohlenstoffatom kein Bestandteil einer Carboxylfunktion oder eines aromatischen Rings ist.

Es gibt also chemische oder physikalische Änderungen der Strukturen des Oxidoreduktase-Zentrums des MIFs, das heißt Alkylierungen wie auch weitere kovalente Modifikationen sowie Bindungen an Kohlenhydrat- und Lipidstrukturen oder Veränderungen bestimmter Bindungen. Die Bereitstellung von bisher nicht bekannten MIFARs weist gegenüber den zum Stand der Technik gehörenden MIF und Cytokin-basierten Therapie- und Diagnosestrategien bei humanen Krankheiten eine Vielzahl von Vorteilen auf. Die therapeutisch und diagnostisch einsetzbaren Substanzen basieren auf der Oxidoreduktaseaktivität von MIF. Da diese in direktem Zusammenhang mit der immunologischen oder physiologischen Wirkung von MIF im Gesamtzusammenhang des betreffenden Körpers steht, können mit den erfindungsgemäßen MIFARs sowohl enzymatische als auch rezeptorvermittelte, cytokinische MIF-Effekte erzielt werden. Zum Beispiel können MIFARs Cysteinthiolgruppen auf MIF-Rezeptoren oder allgemein an mit MIF interagierenden Proteinen (MIF-Bindungsproteinen) wie dem Jabl umsetzen und so eine Zellaktivierung oder - desaktivierung bewirken. Vorzugsweise werden mit MIFARs klassische enzymatische Zielsubstrate von MIF wie auch Cytokinrezeptoren oder andere mit MIF interagierende Proteine als molekulare Zielstrukturen angesprochen, wodurch das Einsatzgebiet für MIFARs viel umfassender ist, als für die im Stand der Technik beschriebenen MIF-basierten Therapeutika.

Erfindungsgemäße MIFARs fallen in ein breites Substanzspektrum: es sind Peptide, modifizierte oder stabilisierte Peptide, cyclische Peptide, DNAs die krankheits- oder patientengruppenspezifisch entwickelt werden können. Darüber hinaus können MIFARs als Gerüste/Templates zur Synthese von weiteren kleinmolekularen Substanzen, zum Beispiel durch Drug Screens, dienen. MIFARs basieren vorzugsweise auf demselben Wirkprinzip, können jedoch stofflich je nach Einsatzgebiet zweckmäßig angepaßt werden. Da ein Großteil der MIFARs chemisch-synthetisch hergestellt werden kann, können so zusätzliche "custom-made"-Anforderungen erfüllt werden.

Die chemische Herstellung von MIFARs garantiert neben der Möglichkeit zur maßgeschneiderten Substanzvariabilität eine Reihe von herstellungs- und lagerungstechnischen Vorteilen gegenüber MIF-Proteinen oder Antikörpern, die rekombinant hergestellt sind.

Ein weiterer wesentlicher Vorteil ist, dass ein Teil der MIFARs chemisch stabilisiert werden kann. Hierdurch wird die metabolische und Proteasestabilität gegenüber MIF oder anderen Derivaten aus dem Stand der Technik entscheidend erhöht. MIFARs haben eine höhere spezifische biologische Aktivität als MIF selbst oder die bekannten Varianten des MIFs. Dies ist vor allem in der Einführung der spezifischen, redoxbasierten chemischen Modifizierungen oder gentechnischen Veränderungen begründet.

Erfindungsgemäße MIFARs, insbesondere die Proteinanaloga, werden durch chemische und biochemische Modifizierungen, zum Beispiel durch Methylierung bestimmter Nα-Atome oder mittels Mutationen gezielt als Monomere hergestellt. Diese sind wesentlich aktiver als die im Stand der Technik bekannten Trimere (Sun et al., 1996), die nach den bisherigen Erkenntnissen die physiologischen, aktiven Formen darstellen. Dadurch sind MIFARs für ein breiteres Einsatzgebiet geeignet als die im Stand der Technik bekannten MIF-basierten Stoffe. So sind MIFARs unter anderem anwendbar bei diversen Immunerkrankungen, bei Krebserkrankungen oder Streß-assoziierten Krankheitsbildern (zum Beispiel allen Krankheiten, die durch oxidativen Streß verursacht werden).

Es hat sich gezeigt, dass MIFARs eine geringe Antigenität aufweisen. Dies gilt insbesondere für die erfindungsgemäß vorgesehenen kleinen MIF-Peptide aus 16 Aminosäuren und ihre modifizierten Derivate.

Ein weiterer Vorteil ist, dass MIFARs effektive Capturemoleküle oder spezifische Kompetitoren oder Templatstrukturen bei Screeningverfahren darstellen, zum Beispiel bei Verfahren, insbesondere HTP-Verfahren (hight through put) zur Identifizierung von potentiellen pharmazeutisch wirksamen Substanzen, insbesondere Verfahren zur Untersuchung der Interaktion von Makrophagen-Migrationsinhibitionsfaktor (MIF) mit Zielproteinen oder -strukturen wie Jab 1 unter Anwesenheit und Abwesenheit von der zu testenden Substanz. In einem solchen Verfahren können die vorliegenden MIFARs als vierte Komponente hinzugegeben werden, um so die Wirkung der zu testenden Substanz auf die Interaktion von (Wildtyp) MIF mit Jab 1 oder anderen Interaktoren unter Einfluss eines zu MIF kompetitiven Agens genauer untersuchen zu können. Ein entscheidender Vorteil der erfindungsgemäßen Lehre ist, dass man mit MIFARs zugleich auf kleinmolekulare Substrate zum Beispiel für die Enzymaktivität, aber auch auf Rezeptoren, Bindungsproteine und ähnliches screenen kann.

Für die Diagnose werden aufgrund ihres biologischen Wirkungsgrades mit MIFARs zum Beispiel neue effektive Sonden, Kompetitoren (zum Beispiel für Aktivitäts- und Screeningsassays, die nach dem Prinzip der Kompetition funktionieren, zum Beispiel kompetitive ELISAs oder kompetitive Bindungsassays) bereitgestellt.

Da die erfindungsgemäßen MIFARs die immunologische Wirkung von MIF antagonisieren, hemmen oder mimikrieren, werden mit der Erfindung neue spezifische und hochwirksame Substanzen für effektive MIF-basierte Therapie-, Diagnose- und Screeningansätze bereitgestellt. Erfindungsgemäß können aus der Kombination von Redox- und Cytokinaktivität beziehungsweise in bestimmten Fällen durch die Redoxaktivität allein medizinische Anwendungen resultieren.

Je nach Art des Derivates, der Modifizierung, der Mutation und/oder chemischen Modifikation handelt es sich bei den MIF-Protein/Peptidanaloga sowohl um Agonisten als auch um Antagonisten oder Modulatoren.

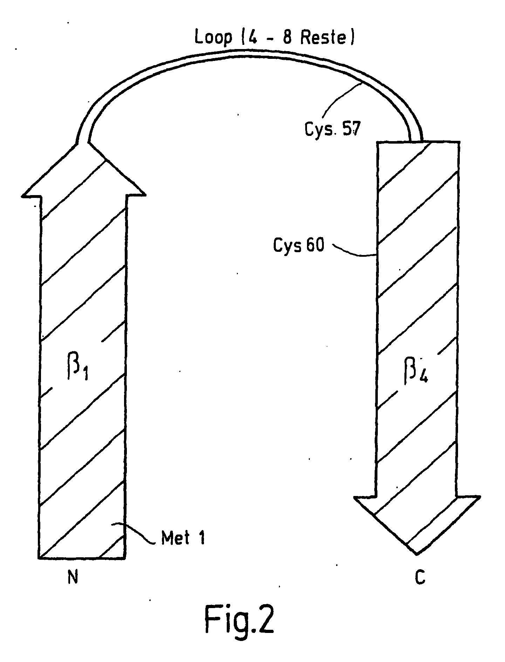

In einer besonderen Ausführungsform ist der MIF ein MIF aus Mensch, Ratte, Maus. Die MIFs aus Mensch, Ratte und Maus sind mit einer Identität von 90% im Sinne der Erfindung als homolog zu bezeichnen, da insbesondere der Bereich um das Oxidoreduktase-Zentrum und das Oxidoreduktase-Zentrum selbst identisch sind. Zwischen der Aminosäureposition 50 und 65 sind bei Mensch und Maus nur 4 Aminosäuren unterschiedlich, wobei die Austausche konservativer Natur sind. Der MIF der Ratte und der Maus sind identisch bis auf den Austausch einer einzigen Aminosäure, der jedoch nicht Cys betrifft. Sofern nicht anders angegeben, beziehen sich im Zusammenhang mit der vorliegenden Erfindung Positionsangaben von Aminosäuren auf die Position der jeweiligen Aminosäure im natürlichen, humanen Wildtyp-MIF, wie in Kleemann et al., 1998b beschrieben, wobei dessen Offenbarungsgehalt hinsichtlich der dort beschriebenen natürlichen Aminosäuresequenz (Figur 2 von Kleemann et al., 1998b) des humanen MIFs vollständig in die vorliegende Offenbarung mit einbezogen ist.

In einer weiteren besonderen Ausführungsform ist das Derivat eines MIFs dadurch ausgezeichnet, dass die Aminosäuresequenz des Oxidoreduktase-Zentrums und/oder die für die Trimerisierung des MIFs notwendigen Aminosäuren modifiziert sind.

In einer nicht erfindungsgemäßen Ausführungsform ist das Derivat eines MIFs mit einer modifizierten Oxidoreduktaseaktivität ein Derivat, das in monomerer Form vorliegt, also unter natürlichen Bedingungen nicht zur üblichen Trimerisierung befähigt ist, und bei dem mindestens eine der Aminosäuren Va140, His41, Gln46, Ala49, Ile97 und Gly108 des MIFs durch eine andere Aminosäure ersetzt ist und/oder alkyliert, insbesondere methyliert, besonders bevorzugt Nα-methyliert, vorliegt. Dies sieht also die Modifikation, das heißt Aminosäureaustausch oder Alkylierung, insbesondere Methylierung der Aminosäuren des MIFs vor, die für die Trimerisierung des MIFs notwendig sind. Offenbart sind auch solche Derivate, bei denen die Aminosäure Val40, His41, Gln46, Ala49, Ile97 oder/und Gly108 des Wildtyp-MIFs durch eine Nα-methylierte Aminosäure ersetzt ist.

In einer nicht erfindungsgemäßen Ausführungsform sind die Derivate eines MIFs die monomeren Derivate, bei denen mindestens eine der Aminosäuren des MIFs an den Positionen Val40, Ala49, Ile97 und/oder Gly108 durch die Aminosäure Pro ersetzt ist. Vorteilhafte Beispiele sind das Derivat von MIF, bei dem der Aminosäurerest Ala49 durch Pro ersetzt ist (das sogenannte MonoMIF-Rekombein-1) oder das Derivat von MIF, bei dem die Aminosäurereste Ala49 und Gly108 durch Pro ersetzt sind (MonoMIF-Rekombein-2), das Derivat von MIF, bei dem der Aminosäurerest Gly108 durch Pro ersetzt ist (MonoMIF-Rekombein-3), das Derivat von MIF, bei dem die Aminosäurereste Ala49 und Val40 durch Pro ersetzt sind (MonoMIF-Rekombein-4) und das Derivat von MIF, bei dem die Aminosäurereste Ile97 und Gly108 durch Pro ersetzt sind (MonoMIF-Rekombein-5). Die offenbarte Lehre bezieht sich selbstverständlich auf alle weiteren Substitutionen an den Positionen Va140, Ala49, Ile97 und/oder Gly108 durch die Aminosäure Pro sowie alle weiteren Substitutionen an den Positionen Val40, Ala49, Ile97 und/oder Gly108 durch andere Aminosäuren als Pro.

In einer weiteren nicht erfindungsgemäßen Ausführungsform betrifft die Lehre ein monomeres Derivat eines MIFs, wobei mindestens eine der Aminosäuren des MIFs an den Positionen His41 und Gln46 durch Ala, Gly und/oder Ser ersetzt ist. So können zum Beispiel bei einem Derivat von MIF die Aminosäurereste His41 und Gln46 durch Ala, Gly oder/und Ser ersetzt sein, wie dies beispielsweise bei MonoMIF-Rekombein-6 der Fall ist. Offenbart sind auch Mono-MIF-Rekombeine, bei denen zusätzlich die Cysteine wie in den MIF-Triple-Muteinen-1-5, im MIF-Triple-Chemoin-1, in den Cyclo-MIF-Syntheinen-1-96 oder/und in den MIF-Syntheinen-1-7 modifiziert sind. Die Definitionen dieser MIF-Varianten sind in den nachfolgenden Absätzen beschrieben.

In einer weiteren nicht erfindungsgemäßen Ausführungsform betrifft die Lehre das Derivat eines MIFs Derivate, wobei mindestens eine der Aminosäuren des MIFs an den Positionen Val40, Ala49, Ile97 und/oder Gly108 methyliert, insbesondere Nα-methyliert ist. Durch diese Methylierung können sehr potente MIF-Agonisten, Antagonisten beziehungsweise Modulatoren gewonnen werden. Beispiele sind chemisch hergestellte MIF-Derivate wie das MonoMIF-Synthein-1, bei dem der Aminosäurerest Ala49 Nα-methyliert ist, das MonoMIF-Synthein-2, bei dem die Aminosäurereste Ala49 und Gly108 Nα-methyliert sind, das MonoMIF-Synthein-3, bei dem der Aminosäurerest Gly108 Nα-methyliert ist, das MonoMIF-Synthein-4 und MonoMIF-Synthein-5, bei denen die Aminosäurereste Ala49 und Val40 beziehungsweise die Aminosäurereste Ile97 und Gly108 Nα-methyliert sind. Diese Modifikationen können mit anderen Modifikationen in Kombination vorliegen. Zum Beispiel können zusätzlich Modifikationen wie in den MIF-Triple-Muteinen-1-5, im MIF-Triple-Chemoin-1, in den Cyclo-MIF-Syntheinen-1-96 oder/und in den MIF-Syntheinen-1-7 vorliegen.

Die Erfindung betrifft auch ein Derivat eines MIFs, wobei mindestens eine, vorzugsweise beide der Aminosäuren Cys 57 und Cys 60 des MIFs durch mindestens eine andere Aminosäure, zum Beispiel auch eine unnatürliche Aminosäure, ersetzt ist, alkyliert ist und/oder die Aminosäure Cys 57 und Cys 60 von einer gemeinsamen Brücke umschlossen und loopstabilisiert vorliegen. Selbstverständlich kann das so modifizierte MIF auch ein gemäß der vorliegenden Erfindung geändertes MIF, zum Beispiel ein monomeres MIF und/oder ein MIF mit einer Brücke sein. Bei diesen erfindungsgemäßen MIFARs handelt es sich um die sogenannten MIF-Syntheine und Cyclo-MIF-Syntheine. Nicht erfindungsgemäß kann auch Cys 81 des MIFs durch mindestens eine andere Aminosäure ersetzt sein oder alkyliert sein.

In einer bevorzugten Ausführungsform betrifft die Erfindung Derivate des MIFs, wobei mindestens eine der Aminosäuren des MIFs an den Positionen Cys57, Cys60 und/oder nicht erfindungsgemäß Cys81 durch Ala, Phe, Tyr und/oder Trp ersetzt ist. Die so erzeugten Varianten werden als MIF-Muteine beziehungsweise MIF-Triple-Muteine bezeichnet. Da die Cysteine, insbesondere an den Positionen 57, 60 und 81, für die Wirkungsweise des natürlichen MIFs von großer Bedeutung sind, wurden diese Cysteinreste vorteilhafterweise durch andere Aminosäuren substituiert. Durch diese Modifikation können die Derivate insbesondere eine potente antagonisierende Wirkung bekommen. Sie können somit als Antagonisten bei der Co-Applikation in der Glucocorticoid-Therapie (bei rheumatoider Arthritis oder multipler Sklerose) zum Einsatz kommen. Da sie endogenes MIF hemmen und somit den endogenen Gegenspieler der Glucocorticoide abschwächen, kann die Therapie vorteilhafterweise mit einer viel geringeren Dosis an Glucocorticoiden durchgeführt werden, was zu wesentlich geringeren Nebenwirkungen führt. Die erfindungsgemäßen Derivate des MIFs sind somit auch als Leitstruktur für potente Antagonisten der MIF-Aktivität anzusehen. In nicht erfindungsgemäßer Ausführungsform sind beim MIF-Triple-Mutein-2 alle drei Cys gegen Ala ausgetauscht, bei MIF-Triple-Mutein-3 alle drei Cys gegen Phe ausgetauscht, bei MIF-Triple-Mutein-4 alle drei Cys gegen Trp ausgetauscht und bei MIF-Triple-Mutein-5 alle drei Cys gegen Tyr ausgetauscht.

In einer weiteren bevorzugten Ausführungsform wird das Derivat eines MIFs dadurch erhalten, dass mindestens eine, vorzugsweise alle drei der Aminosäuren des MIFs an den Positionen Cys57, Cys60 und/oder Cys61 alkyliert ist. Bei dem erfindungsgemäßen MIF-Triple-Chemoin-1 beispielsweise führt die Alkylierung aller drei Cys dazu, dass dieses Derivat nicht nur immunologisch nicht mehr aktiv ist, sondern eine negative Aktivität aufweist, das heißt, es hemmt die Aktivität des natürlichen endogenen MIFs. Daher kann die Struktur ebenfalls eine Leitstruktur für Antagonisten des MIFs umfassen. Bei einer Vielzahl von neuen Medikamenten wird der klinische Einsatz dadurch eingeschränkt, dass kein Antagonist für die zu untersuchenden Stoffe zur Verfügung gestellt werden kann. Es ist für bestimmte Therapien erforderlich, mit dem neuen Medikament ebenfalls einen entsprechenden Hemmstoff zur Verfügung zu stellen, um eventuell auftretende Überreaktionen rechtzeitig verhindern und eliminieren zu können. Die genannten Antagonisten bereichern somit die Anwendungsgebiete der Derivate des MIFs.

Aber diese Hemmstoffe sind nicht nur in der Lage, Derivate in ihrer Wirkung zu minimieren, sondern sie sind vorteilhafterweise auch in der Lage, das natürliche endogene MIF in Patienten zu hemmen. Für mehrere Therapien eröffnen sich somit völlig neue Möglichkeiten, da auf unkomplizierte Weise die Wirkung eines endogenen Cytokins, mit seinen vielfältigen immunologischen Auswirkungen, stark verändert werden kann.

In einer weiteren bevorzugten Ausführungsform werden die Derivate des MIFs dadurch bereitgestellt, dass mindestens eine der Aminosäuren des MIFs an den Positionen Cys57 und/oder Cys60 durch eine unnatürliche Aminosäure ausgetauscht wird. Diese erfindungsgemäßen Derivate, die MIF-Syntheine genannt werden, basieren unter anderem auf der Beobachtung, dass durch den vorteilhaften chemosynthetischen Austausch von einem, zwei oder drei Cys durch unnatürliche Aminosäuren, insbesondere Aminosäuren mit Ähnlichkeit zu Alkylierungsreagenzien, potente Agonisten, Antagonisten beziehungsweise Modulatoren des MIFs gewonnen werden.

In einer weiteren Ausführungsform werden diese Derivate des MIFs dadurch gewonnen, dass die unnatürliche Aminosäure eine Aminosäure mit struktureller Seitenkettenähnlichkeit zu Vinylpyridin oder DTNB oder einer ähnlichen Substanz ist. So werden beispielsweise bei MIF-Synthein-1 (C57X/C60X/C81X-MIF) alle drei Cys bei der chemischen Synthese gegen eine unnatürliche Aminosäure mit struktureller Seitenkettenähnlichkeit zu Vinylpyridin oder DTNB oder einer ähnlichen Substanz ausgetauscht, bei MIF-Synthein-2 (C57X-MIF) wird Cys 57 gegen eine unnatürliche Aminosäure mit strukureller Seitenkettenähnlichkeit zu Vinylpyridin oder DTNB ausgetauscht, bei MIF-Synthein-3 (C60X-MIF) wird Cys 60, bei MIF-Synthein-4 (C81X-MIF) Cys 81, bei MIF-Synthein-5 (C60X/C81X-MIF) Cys 60 und 80 gegen eine unnatürliche Aminosäure mit struktureller Seitenkettenähnlichkeit zu Vinylpyridin oder DTNB ausgetauscht, bei MIF-Synthein-6 (C57X/C81X-MIF) werden Cys 57 und 81 und bei MIF-Synthein-7 (C57X/C60X-MIF) werden Cys 57 und 60 gegen eine unnatürliche Aminosäure X mit struktureller Seitenkettenähnlichkeit zu Vinylpyridin oder DTNB ausgetauscht.

In einer weiteren Ausführungsform sieht die Erfindung vor, dass die Aminosäuren Cys 57 und Cys 60 des natürlichen oder erfindungsgemäß modifizierten MIF von einer gemeinsamen, chemisch kovalent gebundenen Brücke, zum Beispiel einer Lactam-Brücke, umschlossen werden und dadurch loopstabilisiert sind. Diese MIFARs werden als Cyclo-MIF-Syntheine bezeichnet. Selbstverständlich können Cys 57 und/oder Cys 60 auch durch eine andere Aminosäure, wie Ala, Phe, Trp und/oder Tyr oder eine unnatürliche Aminosäure, ersetzt sein oder alkyliert vorliegen.

Durch die Cyclisierung werden ähnlich wie bei der Alkylierung potente MIF-Agonisten, Antagonisten c-der Modulatoren des MIFs gewonnen.

Gemäß einer bevorzugten Ausführungsform werden die Derivate des MIFs die Cyclo-MIF-Syntheine durch chemische Cyclisierung mit einer Brücke zwischen den Aminosäuren an den Positionen 56 und 61 des MIFs gebildet.

In einer weiteren bevorzugten Ausführungsform weisen die Derivate des MIFs, die durch eine Brücke chemisch cyclisiert sind, an der Position 56 des MIFs die Aminosäure Asp, Lys, Orn (Ornithin), Dab (2,4 Diaminobuttersäure), Dap (2,3 Diaminopropionsäure) oder Glu und an der Position 61 die Aminosäure Asp, Lys, Orn, Dab, Dap oder Glu auf. Diese MIF-Derivate können vorteilhafterweise chemisch hergestellte MIF-Derivate sein, bei denen die Region um Cys 57 und 60 durch die Einführung einer Brücke, zum Beispiel einer Lactambrücke, loopstabilisiert ist. Durch die Cyclisierung zwischen den Positionen 56 und 61 und dem Austausch der Aminosäuren an genau diesen Positionen können vorteilhafte Derivate bereitgestellt werden, die immunologisch sehr aktiv sind. Nicht erfindungsgemäße Beispiele werden im folgenden dargestellt. Bei Cyclo-MIF-Synthein-1 erfolgt die chemische Cyclisierung zwischen den Aminosäuren Lys56 an der Position i und Asp61 an i+5 von MIF. Bei Cyclo-MIF-Synthein-2 erfolgt die Cyclisierung zwischen den Aminosäuren Asp56 an der Position i und Lys61 an i+5 von MIF. Bei Cyclo-MIF-Synthein-3 erfolgt die chemische Cyclisierung zwischen den Aminosäuren Orn56 an der Position i und Asp 61 an i+5 von MIF. Bei Cyclo-MIF-Synthein-4 erfolgt die chemische Cyclisierung zwischen den Aminosäuren Asp56 an der Position i und Orn61 an i+5 von MIF. Bei Cyclo-MIF-Synthein-5 erfolgt die chemische Cyclisierung zwischen den Aminosäuren Dab56 an der Position i und Asp61 an i+5 von MIF. Bei Cyclo-MIF-Synthein-6 erfolgt die chemische Cyclisierung zwischen den Aminosäuren Asp56 an der. Position i und Dab61 an i+5 von MIF. Bei Cyclo-MIF-Synthein-7 erfolgt die chemische Cyclisierung zwischen den Aminosäuren Dap56 an der Position i und Asp61 an i+5 von MIF. Bei Cyclo-MIF-Syntein-8 erfolgt die chemische Cyclisierung zwischen den Aminosäuren Asp56 an der Position i und Dap61 an i+5 von MIF. Bei Cyclo-MIF-Synthein-9 erfolgt die chemische Cyclisierung zwischen den Aminosäuren Lys56 an der Position i und Glu61 an i+5 von MIF. Bei Cyclo-MIF-Synthein-10 erfolgt die chemische Cyclisierung zwischen den Aminosäuren Glu56 an der Position i und Lys61 an i+5 von MIF. Bei Cyclo-MIF-Synthein-11 erfolgt die chemische Cyclisierung zwischen den Aminosäuren Orn56 an der Position i und Glu61 an i+5 von MIF. Bei Cyclo-MIF-Synthein-12 erfolgt die chemische Cyclisierung zwischen den Aminosäuren Glu56 an der Position i und Orn61 an i+5 von MIF. Bei Cyclo-MIF-Synthein-13 erfolgt die chemische Cyclisierung zwischen den Aminosäuren Dap56 an der Position i und Glu61 an i+5 von MIF. Bei Cyclo-MIF-Synthein-14 erfolgt die chemische Cyclisierung zwischen den Aminosäuren Dap56 an der Position i und Dap61 an i+5 von MIF. Bei Cyclo-MIF-Synthein-15 erfolgt die chemische Cyclisierung zwischen den Aminosäuren Dap56 an der Position i und Dap61 an i+5 von MIF. Bei Cyclo-MIF-Synthein-16 erfolgt die chemische Cyclisierung zwischen den Aminosäuren Glu56 an der Position i und Dap61 an i+5 von MIF. Ein besonderer Vorteil ist, dass diese Stoffe durch die chemische Stabilisierung der Loopregion MIF-Agonisten mit höherer Redox-Aktivität sind.

Gemäß einer weiteren bevorzugten Ausführungsform der Erfindung liegt bei den Derivaten des MIFs eine Brücke zwischen den Aminosäuren an den Positionen 55 und 62 vor.

Gemäß einer bevorzugten Ausführungsform wird das natürliche oder erfindungsgemäß modifizierte MIF dergestalt abgewandelt, dass es an der Position 55 des MIFs Asp, Lys, Orn, Dap, Dab oder Glu und an der Position 62 die Aminosäure Asp, Lys, Orn, Dap, Dab oder Glu aufweist. Nicht erfindungsgemäße Beispiele werden im folgenden dargestellt. Die MIF-Derivate Cyclo-MIF-Synthein-17 bis Cyclo-MIF-Synthein-24 sind chemisch hergestellte MIFs, bei denen die Region um Cys 57 und 60 durch die Einführung einer Brücke, zum Beispiel einer Lactambrücke, loopstabilisiert ist und die chemische Cyclisierung zwischen den für die Cyclo-MIF-Syntheine-1 bis Cyclo-MIF-Syntheine-16 genannten Aminosäuren an der Position i und i+7 von MIF, also an den Positionen 55 und 62, erfolgt. Von Vorteil ist es, diese Stoffe als MIF-Agonisten mit höherer Redox- und Immunaktivität einzusetzen.

Die Cyclo-MIF-Synthein-25 bis Cyclo-MIF-Synthein-96 sind chemisch hergestellte MIFs, bei denen Cys 57 und/oder 60 durch Ala und/oder Gly ersetzt sind, die Region um die Aminosäurereste 57 und 60 durch Einführung einer .Brücke; zum Beispiel einer Lactambrücke, loopstablisiert sind und die chemische Cyclisierung zwischen den Aminosäuren wie für die Cyclo-MIF-Syntheine-1-24 beschrieben erfolgt. Die Loopregion wird bei diesen Stoffen um die ersetzten Aminosäurereste 57 und 60 gebildet. Diese Stoffe weisen vorteilhafterweise als MIF-Antagonisten geringere Redox- und somit geringere immunologische Aktivität auf. Selbstverständlich können die erfindungsgemäßen Derivate auch zwei oder mehrere der vorgenannten Brücken aufweisen.

In einer besonders bevorzugten Ausführungsform betrifft die Erfindung Derivate natürlichen oder erfindungsgemäß modifizierten MIFs, die aus 16 Aminosäuren bestehen und die Aminosäuren Cys-X-X-Cys, insbesondere Cys-Ala-Leu-Cys aufweisen. Nicht erfindungsgemäße Derivate können auch aus 11 bis 15 Aminosäuren bestehen.

Gemäß der Erfindung besteht das Derivat eines MIFs aus 16 Aminosäuren und ist ausgewählt aus der Gruppe bestehend aus
a) einem Derivat eines MIFs, wobei das Derivat die Aminosäuresequenz 50-65 oder 51-66 des Wildtyp-MIFs aufweist und
b) einem Derivat eines MIFs, wobei das Derivat die Aminosäuresequenz 50-65 oder 51-66 des Wildtyp-MIFs aufweist und 1 bis 5 Aminosäuren ausgetauscht sind.
   Nicht zur Erfindung gehörend sind Derivate ausgewählt aus der Gruppe bestehend aus
   - einem Derivat eines MIFs, wobei das Derivat 11-15 Aminosäuren mit der Aminosäurensequenz 51-65, 52-65, 53-65, 54-65, 55-65 des Wildtyp-MIFs aufweist
   - einem Derivat eines MIFs, wobei das Derivat 11-15 Aminosäuren mit der Aminosäurensequenz 51-65, 52-65, 53-65, 54-65, 55-65 des Wildtyp-MIFs aufweist und 1 bis 5 Aminosäuren ausgetauscht sind und
   - einem Derivat eines MIFs, wobei das Derivat eine Kombination zweier oder mehrerer der genannten Derivate und einer Cyclisierung darstellt und 1 bis 5 Aminosäuren ausgetauscht sind.

Die vorgenannten erfindungsgemäßen MIF-Redoxpeptide beruhen auf den überraschenden Befunden, dass bestimmte
(i) kurze MIF-Peptide eine reduzierbare Disulfidbrücke ausbilden, enzymatisch aktiv sind, und immunologische MIF-Aktivität aufzeigen, sowie MIF-artige Interaktionen mit MIF-Bindungsproteinen eingehen können und die sogenannten
(ii) P-Peptide dagegen biologisch nicht aktiv sind und deshalb als Hemmstoffe eingesetzt werden können.

Beispielsweise beinhaltet MIF-Pep-16₁, ein 16 aminosäurelanges Peptidfragment von MIF, die Sequenz 50-65 des humanen MIFs; MIF-Pep-16₂, ein 16 aminosäurelanges Peptidfragment von MIF, beinhaltet die Sequenz 51-66 des humanen MIFs; MIF-Pep-16₃, ein 16 aminosäurelanges Peptidfragment von MIF, welches die Sequenz 50-65 oder 51-66 beinhaltet und bei dem 1 bis 5 Aminosäuren nach Standardmethoden durch andere Aminosäuren ausgetauscht sind; die nicht erfindungsgemäßen MIF-Pep-11 bis MIF-Pep-15 sind 11 bis 15 aminosäurenlange Peptidfragmente von MIF, welche die Sequenzen 51-65, 52-65, 53-65, 54-65, 55-65 beinhalten und bei denen gegebenenfalls in einer Ausgestaltung gegebenenfalls 1 bis 5 Aminosäuren nach Standardmethoden durch andere Aminosäuren ausgetauscht sein können. Erfindungsgemäße P-Peptide sind vorteilhafterweise die P16er Muts, 16 aminosäurelange Peptidfragmente von MIF, welche die Sequenz 50-65 oder 51-66 beinhalten und bei denen Cys 57 und/oder 60 durch Ala und/oder Gly ausgetauscht sind und bei denen gegebenenfalls in einer weiteren bevorzugten Ausführungsform 1 bis 5 Aminosäuren nach Standardmethoden durch andere Aminosäuren ausgetauscht sind. Die nicht erfindungsgemäßen P11erMuts bis P15erMuts sind 11 bis 15 aminosäurelange Peptidfragmente von MIF, welche Sequenzen wie die bereits angeführten beinhalten und bei denen Cys 57 und/oder 60 gegen Ala und/oder Gly ausgetauscht sind und bei denen gegebenenfalls in einer weiteren bevorzugten Ausführungsform 1 bis 5 Aminosäuren nach Standardmethoden durch andere Aminosäuren ausgetauscht sind. MIF-Cyclo-Peps sind vorteilhafte Varianten von MIF-Pep-16, MIF-Pep-11 bis MIF-Pep-15 oder PxMuts, bei denen eine Cyclisierung benachbarter Aminosäurereste, zum Beispiel durch Lactamverbrückung, durchgeführt wurde und bei denen gegebenenfalls in einer weiteren bevorzugten Ausführungsform 1 bis 5 Aminosäuren nach Standardmethoden durch andere Aminosäuren ausgetauscht sind.

Im Zusammenhang mit der vorliegenden Erfindung ist ein Austausch der Aminosäuren nach Standardmethoden ein Aminosäureaustausch vor, hinter und auch innerhalb der CXXC-Region an den Positionen 57 bis 60 beziehungsweise diesen Positionen entsprechenden Stellen durch andere, ähnliche, natürliche Aminosäuren, insbesondere ein konservativer Aminosäureaustausch, also ein Austausch durch strukturähnliche Aminosäuren; zum Beispiel den konservativen Austausch von Glu durch Asp. Es sind jedoch auch Austausche durch strukturähnliche unnatürliche Aminosäuren zum Beispiel Lys durch Orn, D-Aminosäuren oder N-Methylierungen möglich. Vorteilhaft ist auch der Austausch von Cys selbst zum Beispiel durch andere Thiol-Aminosäuren. Insbesondere bei den P-11erMuts bis P-16erMuts sowie den MIF Cyclo-Peps wurde Cys gegen andere Aminosäuren nach Standardmethoden ausgetauscht, zum Beispiel durch größenmäßig äquivalenten Austausch durch Ala.

Die Erfindung betrifft also Derivate eines ein Oxidoreduktasezentrum mit den Aminosäuren N-Terminus .... Cys-Ala-Leu-Cys .... C-Terminus in den Position 57 bis 60 aufweisenden Makrophagen-Migrations-Inhibitions-Faktors (MIF), wobei das Derivat 16 Aminosäuren aufweist und in löslicher Form vorliegt. Ein solches Derivat kann im Oxidoreduktasezentrum in besonders bevorzugter Ausführungsform anstelle von Cystein an Position 57 und/oder 60 des Wildtyp-MIF, das heißt an der entsprechenden Position im Peptid, Serin aufweisen, das heißt insbesondere die Aminosäuresequenz N-Terminus .... Cys-Ala-Leu-Ser.... C-Terminus, N-Terminus .... Ser-Ala-Leu-Cys ... C-Terminus oder N-Terminus.... Ser-Ala-Leu-Ser.... C-Terminus.

In einer weiteren nicht erfindungsgemäßen Ausführungsform beschreibt die vorliegende Lehre auch Derivate eines ein Oxidoreduktasezentrum mit den Aminosäuren N-Terminus .... Cys-Ala-Leu-Cys .... C-Terminus in den Position 57 bis 60 aufweisenden Makrophagen-Migrations-Inhibitions-Faktors (MIF), wobei die Derivate 21 bis 25 Aminosäuren aufweisen und in löslicher Form vorliegen und wobei die Aminosäuresequenz des β1-Stranges von MIF über eine 4 bis 8 Aminosäure-Reste umfassende Loop-Struktur direkt kovalent mit der Aminosäuresequenz des β4-Strang von MIF verknüpft ist.

In nativem MIF wird der β1-Strang von den Resten Pro-2 bis Thr-8 gebildet. Der β1-Strang ist ein integraler Bestandteil des zentralen viersträngigen β-Faltblattes von MIF. Das β-Faltblatt stellt nicht nur den Kernbestandteil des MIF-Monomers dar, sondern ist auch an der Oligomerisierung der Untereinheiten durch Wechselwirkung mit den benachbarten Untereinheiten beteiligt. Der β1-Strang bildet mit dem β4-Strang eine antiparallele Faltblatt-Struktur aus, wobei die Aminosäurereste, die an den katalytischen Aktivitäten von MIF beteiligt sind, in enge räumliche Nähe zueinander gebracht werden. Der β1-Strang umfaßt dabei die Bindungsstelle für das Tautomerase-Substrat, während der β4-Strang zusammen mit den beiden vorausgehenden Aminosäure-Resten das CXXC-Motiv enthält, das für die Redox-Aktivität von MIF erforderlich ist.

Die im folgenden als β1/β4-Hairpin-Mini-MIF bezeichneten Derivate sind dadurch gekennzeichnet, dass sie eine Mini-β-Faltblatt-Struktur ausbilden. Diese Derivate können sowohl die TautomeraseAktivität als auch die beiden bekannten Oxidoreduktase-Aktivitäten von MIF, das heißt die 2-Hydroxyethyldisulfid (HED)-Reduktions- und die Insulin-Reduktions-Aktivität, aufweisen. Die β1/β4-Hairpin-Mini-MIF-Derivate besitzen darüber hinaus eine MIF-spezifische immunologische Reaktivität. Die Derivate bieten also in überraschender Weise den Vorteil, dass es sich um kleine Moleküle handelt, die einerseits die Aktivitäten von MIF, also die Tautomerase- und die Oxidoreduktase-Aktivitäten, aufweisen und andererseits alle Stellen besitzen, die für die immunologische Reaktivität von MIF erforderlich sind.

In einer Ausgestaltung umfassen die β1/β4-Hairpin-Mini-MIF-Derivate eine die Aminosäure-Reste Asp und Pro enthaltende Loop-Struktur. Vorteilhafterweise kann die Loop-Struktur dieser Derivate außerdem kleine Aminosäure-Reste, wie beispielsweise Gly, oder Aminosäure-Reste, die häufig in β-Turn-Strukturen gefunden werden und für diese charakteristisch sind, oder Aminosäure-Reste enthalten, die zur Bildung von Lactam-Brücken fähig sind, enthalten. Dazu zählen beispielsweise die Aminosäuren Asp, Glu, Lys und Orn.

Weiterhin betrifft die Erfindung eine Nucleinsäuresequenz, die eines der vorstehend beschriebenen erfindungsgemäßen Derivate eines MIFs kodiert. Mit Hilfe der Nucleinsäuresequenzen ist es vorteilhafterweise möglich, gentechnisch veränderte Organismen herzustellen. Die erfindungsgemäßen Nucleinsäuren können aus natürlichen Quellen isoliert werden oder sie können nach bekannten Verfahren synthetisiert werden. Mittels gängiger molekularbiologischer Techniken ist es möglich, verschiedenartige Mutationen in die erfindungsgemäßen Nucleinsäuren einzufügen, wodurch es zur Synthese von MIFARs mit veränderten biologischen Eigenschaften kommt, die ebenfalls von der Erfindung erfaßt werden. Mutationen im Sinne der Erfindung sind auch Deletionsmutationen, die zu verkürzten MIFARs führen. Durch andere molekulare Mechanismen wie Insertion, Duplikation, Transposition, Inversion, Nucleotid-Austausch oder auch durch Gentransfer kann es beispielsweise zu Modifikationen der biologischen Aktivität der Derivate des MIFs kommen. Auf diese Weise können vorteilhafterweise Derivate des MIFs hergestellt werden, die eine veränderte immunologische oder modulierende beziehungsweise regulierende Aktivität besitzen. Desweiteren können Derivate hergestellt werden, die eine modifizierte Stabilität, Spezifität oder ein verändertes Aktivitäts-, Temperatur-, pH-Wert- und/oder Konzentrationsprofil aufweisen. Weiterhin bezieht sich die erfindungsgemäße Lehre auf Derivate des MIFs, die einen modifizierten Aufbäu möglicher Untereinheiten, prä- und posttranslatiönale Modifikationen und/oder andere Modifikationen aufweisen.

Offenbart sind auch Nucleinsäuren, die mit den vorgenannten erfindungsgemäßen Nucleinsäuren hybridisieren. Hybridisierung bedeutet eine Hybridisierung unter konventionellen Hybridisierungsbedingungen, wie sie in Sambrook et al. (Molecular Cloning. A laboratory manual, Cold Spring Harbor Laboratory Press, 2. Ausgabe, 1989) beschrieben ist, vorzugsweise unter stringenten Bedingungen. Eine Hybridisierung liegt vor, wenn nach Waschen für eine Stunde mit 1 x SSC und 0,1% SDS bei 55°C, vorzugsweise bei 62°C und besonders bevorzugt bei 68°C, insbesondere für 1 Stunde in 0,2 x SSC und 0,1% SDS bei 55°C, vorzugsweise bei 62°C und besonders bevorzugt bei 68°C noch ein positives Hybridisierungssignal beobachtet wird.

Bei den erfindungsgemäßen Nucleinsäuresequenzen kann es sich sowohl um DNA als auch RNA-Moleküle handeln. Die DNA-Moleküle sind beispielsweise genomische oder cDNA-Moleküle.

Die Erfindung betrifft weiterhin einen Vektor, der die erfindungsgemäße Nucleinsäuresequenz enthält.

In einer bevorzugten Ausführungform handelt es sich bei dem Vektor um einen bakteriellen oder viralen Vektor, Plasmid, Bacteriophagen oder um andere in der Gentechnik übliche Vektoren, zum Beispiel auch Liposomen.

Vorzugsweise ist die Nucleinsäuresequenz im erfindungsgemäßen Vektor mit regulatorischen Elementen operativ verbunden, die Transcription und Synthese einer translatierbaren RNA in pro- und/oder eucaryotischen Zellen gewährleisten. Die erfindungsgemäße Nucleinsäuresequenz kann in sense- oder antisense-Orientierung zu diesen regulatorischen Elementen vorliegen. Derartige regulatorische Elemente sind Promotoren, Enhancer oder Transcriptionsterminationssignale, können aber auch Introns oder ähnliche Elemente sein. Im Vektor können selbstverständlich weitere funktionelle Elemente vorhanden sein, wie Elemente, die die Stabilität und Vermehrung des Vektors, die Selektion und/oder die Integration in das Wirtsgenom ermöglichen oder dazu beitragen.

Die Erfindung betrifft auch Wirtszellen, die eine der erfindungsgemäßen Nucleinsäuresequenzen oder einen der erfindungsgemäß beanspruchten Vektoren beinhalten beziehungsweise mit diesen transformiert sind. Weiterhin betrifft die Erfindung alle Wirtszellen, die von einer mit den erfindungsgemäßen Nucleinsäuresequenzen oder den erfindungsgemäßen Vektoren transformierten Wirtszellen abstammen. Die Erfindung betrifft also Wirtszellen, die in der Lage sind, die erfindungsgemäßen Nucleinsäuresequenzen oder Vektoren zu enthalten, wobei unter einer Wirtszelle ein Organismus verstanden wird, der in der Lage ist, Nucleinsäuresequenzen aufzunehmen und gegebenenfalls die von den erfindungsgemäßen Nucleinsäuresequenzen kodierten Derivate des MIFs zu synthetisieren. Vorzugsweise sind dies procaryotische oder eucaryotische Zellen. Die erfindungsgemäße Wirtszelle kann auch dadurch gekennzeichnet sein, dass die eingeführte erfindungsgemäße Nucleinsäuresequenz entweder heterolog in bezug auf eine transformierte Zelle ist, d. h. natürlicherweise nicht in dieser Zelle vorkommt oder aber an einem anderen Ort, einer anderen Orientierung oder in einer anderen Kopiezahl im Genom lokalisiert ist als die entsprechende natürlicherweise auftretende Sequenz.

Die Erfindung betrifft weiterhin eine Zellkultur die mindestens eine Wirtszelle, die mit mindestens einem erfindungsgemäßen Vektor transformiert ist, umfaßt.

Die Erfindung betrifft jedoch auch ein Verfahren zur Herstellung eines erfindungsgemäßen Derivates eines MIFs, wobei die erfindungsgemäße Wirtszelle in einem Kulturmedium unter solchen Bedingungen kultiviert wird, die die Bildung eines erfindungsgemäßen Derivates eines MIFs dergestalt erlauben, dass das Derivat eines MIFs gewonnen werden kann.

In einer weiteren besonderen Ausführungsform ist das erfindungsgemäße Derivat eines MIFs immobilisiert. Erfindungsgemäß besonders bevorzugt erfolgt die Immobilisierung in besonders vorteilhafter Weise über den C-Terminus der MIFARs. Besonders vorteilhaft ist, dass teildenaturiertes MIF-Derivat, wie zum Beispiel nach der Immobilisierung mit manchen Methoden, im Gegensatz zu den bisherigen immobilisierten Cytokinen, noch aktiv ist. Die Immobilisierung an inerte oder elektrisch geladene, anorganische oder organische Trägermaterialien wie zum Beispiel poröse Gläser, Silicalgel, Aluminiumoxid, Cellulose, Stärke, Dextran, Polyacrylamid, Polyvinylalkohol oder Nylon kann mit der Methode der Adsorption erfolgen. Weiterhin ist die Bindung an Trägermaterialien, die reaktive Gruppen aufweisen durch kovalente Bindung möglich. Vorteilhaft ist jedoch auch der Einschluß des erfindungsgemäßen Derivates in dreidimensionale Netzwerke. Hierzu wird die Polymerisation beziehungsweise die Gelantisierung in wäßriger Lösung in Gegenwart der beanspruchten Derivate durchgeführt. Die Immobilisierung kann jedoch auch durch Quervernetzung mit bifunktionellen Reagenzien erfolgen. Außerdem ist es möglich, die Immobilisierung des erfindungsgemäßen Derivates mit Hilfe der Mikroverkapselung durchzuführen. Die Mikroverkapselung kann als Grenzflächenpolymerisation durchgeführt werden.

In einer bevorzugten Ausführungsform ist das erfindungsgemäße immobilisierte Derivat ein Derivat, das in medizinischen Vorrichtungen, drug-delivery-Vorrichtungen, Matrizen für drug-screening-Bibliotheken und/oder in applizierbaren Katalyse-vorrichtung Einsatz findet. Diese medizinische Anwendung ist deshalb möglich, da die erfindungsgemäßen Derivate auch unter den denaturierenden Bedingungen wie sie bei der Immobilisierung eingesetzt werden können, biologisch aktiv bleiben. So ist es vorteilhafterweise möglich, die erfindungsgemäßen MIFARs auf Trägern in immobilierter Form einzusetzen und diese so molekular zu definieren. MIF wirkt immunologisch über seine Redoxaktivität; zur "Rezeptor"-Aktivierung ist monomeres MIF ausreichend, eine Internalisierung ist nicht zwingend erforderlich. MIF ist auch nach seiner Immobilisierung auf einem festen Träger katalytisch redoxaktiv. Besonders zweckmäßig ist es, immobilisierte MIFARs als lokal und ortsspezifisch applizierte Katalyse-Devices zur Regulierung der Redox-Balance des Körpers zur Senkung des oxidativen Stresses sowie zur Tumorunterdrückung einzusetzen. Immobilisierte MIFARs stellen stabile trägergebundene Screening-Devices dar, die zweckmäßigerweise länger gelagert werden können und die auch für automatisierte Screeningverfahren geeignet sind.

Es gibt daher auch Verfahren zum Identifizieren von potentiell als pharmazeutische Wirkstoffe geeigneten Substanzen, das heißt Drug-Screening-Verfahren, wobei die Interaktion von Wildtyp-MIF mit einem seiner Interaktionspartner, zum Beispiel dem Bindungspartner Jab in An- und Abwesenheit eines potentiellen Arzneimittels untersucht wird und wobei die erfindungsgemäßen MIFARs als zum Wildtyp-MIF kompetitive Bindungspartner eingesetzt werden. Selbstverständlich ist es auch möglich, die erfindungsgemäßen MIFARs anstelle des Wildtyp-MIF als Bindungspartner zu einem Bindungspartner des Wildtyp-MIF, zum Beispiel Jab, einzusetzen, und in Anwesenheit dieser beiden Komponenten potentielle Arzneimittel zu identifizieren. Selbstverständlich ist es auch möglich, die erfindungsgemäßen MIFARs zusammen mit Derivaten des natürlichen Bindungspartners des Wildtyp-MIFs, zum Beispiel Jab-Derivaten, in Drug-Screening-Verfahren, das heißt in An- und Abwesenheit potentieller Arzneimittel einzusetzen, das heißt in einem leichter handhabbaren Modellsystem. Besonders bevorzugte MIFARs der vorliegenden Erfindung für die genannten Screeningverfahren sind zum Beispiel MIF 50 bis 65 (gleich MIF-Pep-16₁) oder Ser⁵⁷, Ser⁶⁰ MIF 50 bis 65 (also 16 Aminosäuren langes MIF-Peptid mit Wildtyp-Aminosäuresequenz der Positionen 50 bis 65, wobei die Wildtyp-Cys-Reste an Position 57 und 60 jeweils durch Ser ersetzt sind).

Weiterhin bevorzugt ist die Anwendung der erfindungsgemäßen MIFARs in-vitro Diagnostikkits für die Forschung und Entwicklung. Erfindungsgemäße MIFARs sind vorteilhafterweise der spezifizierende Teil in Substanzscreenings-Assays. Die erfindungsgemäßen Stoffe können ihren therapeutischen, diagnostischen und Screeningeinsatz mit besonderem Vorteil beispielsweise bei der Therapie folgender Krankheiten finden: Multiple Sklerose und andere inflammatorische Erkrankungen des Zentralnervensystems, Krebserkrankungen, Morbus Crohn, inflammatorische Lungenerkrankungen wie das Adult Respiratory Distress Syndrome (ARDS) oder chronisches, akutes und allergisches Asthma, bakterielle und allergische Sepsis, rheumatoide Arthritis und/oder Spättyp-Allergien (DTH). Selbstverständlich ist aber auch eine Anwendung bei weiteren Krankheiten möglich. Da MIF als einziges Cytokin die immunsuppressiven und antiinflammatorischen Wirkungen der Glucocorticoide hemmt, können MIFARs besonders bevorzugt als Co-Therapeutika und diagnostische Tools bei mit Glucocorticoiden behandelten Erkrankungen Verwendung finden. Die erfindungsgemäßen Substanzen stellen daher vorteilhafterweise potentielle MIF-spezifische Therapeutika und Diagnostika dar beziehungsweise dienen als Substanzgerüste zur Entwicklung dieser. Zweckmäßig ist es, diese Substanzen als Therapeutika und Diagnostika bei Krankheiten einzusetzen, bei denen MIF regulatorisch beteiligt ist. Zum Beispiel bieten redoxspezifische MIF-Analoga neue therapeutische Ansätze für die Therapie multipler Sklerose. Agonisten und Antagonisten stellen zum Beispiel aufgrund ihrer biochemischen und immunologischen Eigenschaften auch zweckmäßige Co-Therapeutika bei Steroid-Applikationen bei mit Glucocorticoiden behandelten Krankheiten dar, wo sie die Glucocorticoideffekte abschwächen oder verstärken können. Von Vorteil ist, dass der letztere Fall die Applikation geringerer und somit nebenwirkungsfreierer Glucocorticoidmengen erlaubt. Die erfindungsgemäßen MIFARs sind biochemisch oder chemisch so beschaffen, dass sie therapeutisch und' diagnostisch effektiv applizierbar beziehungsweise einsetzbar sind, da sie beispielsweise chemisch und metabolisch stabil sind.

Dem Anwendungsgebiet liegt unter anderem die Beobachtung zugrunde, dass MIF-Derivate im Körper des Menschen bei Infektionen, chronischen Entzündungskrankheiten oder Allergien als typisches proinflammatorisches Cytokin bei der Entstehung und Pathogenese bestimmter inflammatorischer oder Immunkrankheiten ursächlich oder katalysierend beteiligt ist, für die derzeit keine zufriedenstellende Behandlungs- und Diagnoseverfahren existieren.

In erfindungsgemäßen Screeningverfahren für zum Beispiel potentielle Arzneimittel stellen MIFARs vorzugsweise effektive Capturemoleküle beziehungsweise spezifizierende Kompetitoren und ähnliches dar. Die erfindungsgemäßen MIFARs können beispielsweise als Templates oder Vorbilder für die Entwicklung von Arzneimitteln oder Diagnostika dienen, insbesondere für solche Substanzen, die die spezifische Interaktion, zum Beispiel Bindung, von MIF mit dessen Interaktionspartnern, zum Beispiel Jabl, modifizieren, zum Beispiel reduzieren, unterbinden, fördern oder initiieren. Von Vorteil ist, dass man mit MIFARs zugleich kleinmolekulare Substrate für die Enzymaktivität, aber auch Rezeptoren und Bindungsproteine identifizieren kann. MIFARs haben unter anderem den Vorteil, dass sie biologisch aktiv sind und diese Eigenschaften zudem auch noch nach Immobilisierung besitzen. Daher sind die erfindungsgemäßen Analoga, Antagonisten, Agonisten, Inhibitoren, Varianten und Teilsequenzen vorteilhafterweise in verschiedenen Screening-Assays in Kliniken, ärztlichen Praxen, Forschungseinrichtungen und Unternehmen einsetzbar. In allen Fällen ist das Anwendungsgebiet die Medizin in ihren Teilbereichen Prophylaxe, Krankheitsbehandlung und Stärkung des Immunsystems, letzteres als Einsatzgebiet der Therapeutika, Krankheitsfrüherkennung und Krankheitsdiagnose (diagnostische Screeningtests) sowie Medikamentenfindung (Substanzscreening). Im erster Fall sind die gefundenen Stoffe besonders zweckmäßig als Pharmazeutika einsetzbar, im zweiten Fall sind die Stoffe vorteilhafterweise die molekulare Grundlage neuer diagnostischer Tests oder Testkits.

Die Erfindung betrifft auch polyclonale und monoclonale Antikörper, die eine spezifische Affinität zu dem erfindungsgemäßen Derivat aufweisen. Vorzugsweise handelt es sich bei den Antikörpern um Antagonisten beziehungsweise Hemmstoffe. Die sogenannten redoxspezifischen Anti-MIF-Antikörper zeichnen sich dadurch aus, dass sie spezifisch gegen die für die MIF-Redoxaktivität wichtigen Molekülregionen gerichtet sind. Sie sind daher spezifischer und nativer als "zufällig" diese Region abdeckende Antikörper, da sie nach Immunisierung mit einem bioaktiven MIF-Redoxstoff erzeugt werden können.

Zum Stand der Technik gehörende Anti-MIF-Antikörper werden bisher durch bekannte Techniken zur Erzeugung mono- oder polyclonaler Antikörper hergestellt. Sie wirken daher lediglich durch nichtkovalente Bindungen an "zufällig gefundene" MIF-Epitope. Dabei hat das Screening der Antikörper nur auf Basis von "Bindung" und biologischer Aktivität stattgefunden. Es können also nur Bereiche im MIF-Molekül neutralisiert werden, die für die Rezeptorvermittelte Aktivität verantwortlich sind. Diese sind jedoch nicht bekannt, so dass die bereits bekannten "zufällig" gefundenen neutralisierenden Antikörper entsprechend wenig effektiv sind; beispielsweise polyclonale oder monoclonale Antikörper, die zufällig neben der unbekannten rezeptorbindenden Domäne binden. Die erfindungsgemäßen Redox-spezifischen Anti-MIF-Antikörper hingegen haben insbesondere den Vorteil, dass sie durch gezielte Neutralisierung des redoxaktiven Zentrums von MIF, also von einem oder beiden Cystein(en) im Sequenzbereich 57 bis 60 beziehungsweise der entsprechenden Loop-Struktur, die enzymatischen und rezeptorvermittelten MIF-Wirkungen zugleich und hochspezifisch hemmen können. Antikörper mit hoher spezifischer Aktivität können vorteilhafterweise durch Immunisierung mit kleinen erfindungsgemäßen MIF-Peptiden erzeugt werden, welche die für die Redoxaktivität wichtigen Aminosäuren enthalten und durch den Einbezug weiterer bestimmter Aminosäuren eine hohe Immunogenität besitzen.

Weitere vorteilhafte spezifischere redoxspezifische Anti-MIF-Antikörper werden erfindungsgemäß dadurch gewonnen, dass chemisch loopstabilierte MIF-Peptide zur Immunisierung und Antikörperbildung, eingesetzt werden. Diese für die Immunisierung verwendeten Peptide haben gegenüber anderen im Stand der Technik verwendeten trägergekoppelten Peptiden den Vorteil, dass in ihnen die Antikörperzielstruktur (der "Cystein-Loop") chemisch stabilisiert ist und die Antikörper somit gegen die biologisch relevante immunogene räumliche Struktur generiert werden und nicht, wie sonst bei kleineren Peptiden üblich, gegen strukturell flexible, nicht so immunogene Strukturen.

Erfindungsgemäße MIFARs können krankheits- oder sogar patientengruppenspezifisch entwickelt werden. Diese Entwicklung schlägt sich insbesondere in der Generierung hochspezifischer Antikörper nieder. Die Erfindung betrifft vorzugsweise Antikörper, die in der Lage sind, eine Struktur eines erfindungsgemäßen MIF-Derivates zu identifizieren. Der chemische Charakter dieser Struktur ist hierbei nicht entscheidend, es kann sich um Proteine, Kohlenhydrate wie auch Lipidkomplexe und Glycolipide - die mit dem Derivat eines MIF spezifisch in Beziehung stehen - handeln, dies heißt, beispielsweise auch Pintikörper, die gegen Strukturen gerichtet sind, die als posttranslationelle Modifikationen anzusprechen sind, werden von der Erfindung erfaßt. Von Vorteil ist, dass eine räumliche Elektronenwolkenstruktur mittels Antikörpern so identifiziert werden kann, dass Rückschlüsse auf biologische, chemische und physikalische Eigenschaften und/oder Wirkungsweisen der Derivate des MIFs möglich sind. Antikörper mit einer MIF-Redoxspezifität stellen Stoffe dar, die vorteilhafterweise die Wirkung von MIF in einer breiten Wirkung und gleichzeitig dennoch spezifisch hemmen, da sie durch die Neutralisation des Redoxzentrums von MIF sowohl die enzymatischen wie auch die immunologischen Wirkungen von MIF hemmen.

In einer weiteren besonderen Ausführungsform betrifft die Erfindung auch Antikörper, die mit den erfindungsgemäßen vorgenannten Antikörpern interagieren können.

Weiterhin betrifft die Erfindung vorgenannte Antikörper, wobei diese auf biologischem, chemischem und physikalischem Wege modifiziert sind.

Die Erfindung betrifft auch die Verwendung der Nucleinsäuresequenz oder diese enthaltende Vektoren, Wirtszellen oder die Verwendung der erfindungsgemäßen Derivate zur Herstellung von Arzneimitteln zur Behandlung oder Diagnose der vorgenannten Krankheiten beziehungsweise zur Herstellung eines Arznei- oder Diagnosemittels zur Diagnostik und/oder Therapie von Tumorerkrankungen, onkologischen Erkrankungen sowie von inflammatorischen Erkrankungen und/oder den genannten Indikationen.

In einer besonderen Ausführungsform betrifft die Erfindung Verfahren und Verwendungen unter Einsatz der erfindungsgemäßen MIFARs für die Herstellung von Arzneimitteln zur Diagnose oder Behandlung von inflammatorischen Erkrankungen, insbesondere Erkrankungen des Respirationstraktes, des zentralen Nervensystems und/oder alle allergischen oder davon abgeleiteten Erkrankungen.

In einer besonderen Ausführungsform betrifft die Erfindung Verfahren und Verwendungen unter Einsatz der erfindungsgemäßen MIFARs für die Herstellung von Arzneimitteln zur Diagnose oder Behandlung von karcinogenen oder onkologischen Erkrankungen, Metastasen und Tumoren sowie davon abgeleiteten Erkrankungen.

In einer besonderen Ausführungsform betrifft die Erfindung Verfahren und Verwendungen unter Einsatz der erfindungsgemäßen MIFARs für die Herstellung von Arzneimitteln zur Diagnose oder Therapie von Krankheiten, die mit Glucocorticoiden behandelt werden. Hierbei handelt es sich vorzugsweise um alle die Glucocorticoide, die sich in irgendeiner Form von der Struktur des Prägnan (C₂₁H₃₆) ableiten.

Die Erfindung betrifft also auch die Verwendung der erfindungsgemäßen Derivate des MIFs, weiterhin der erfindungsgemäßen Nucleinsäuren, Vektoren, Wirtszellen, Zellkulturen und/oder der erfindungsgemäßen Antikörper zur Herstellung eines Arzneimittels für die vorgenannten Erkrankungen, insbesondere Tumorerkrankungen oder therapeutische Anwendung bei Krankheiten des Respirationstraktes, des zentralen Nervensystems und/oder allergischen oder davon abgeleiteten Krankheiten.

Die Erfindung betrifft auch die Verwendung der erfindungsgemäßen Antikörper zur Herstellung eines Diagnose- oder Arzneimittels zur Diagnose oder Behandlung der vorgenannten Krankheiten.

### Beispiele

Die Erfindung soll anhand der folgenden Beispiele und der Figuren näher erläutert werden.
Figur 1 stellt einen Anti-MIF IP/Anti-biotin-Westernblot dar.
Figur 2 stellt in schematischer Form die Struktur von β1/β4-Hairpin-Mini-MIF-Derivaten dar.
Figur 3 stellt einen Glucocorticoid-Overriding-Test in der THP-1-Monozytenlinie dar. L+D, positive Kontrolle des Tests: LPS + Dexamethason: entspricht 100% Hemmung; MF2: MIF-Pep-16₁.
Figur 4 stellt dar: 1: Zellen unbehandelt; 2: Kontrolle mit NaPP-Puffer 20 mM; 3: 100 nM rMIF; 4: 500 nM rMIF; 5: 1 µM rMIF; M, Molekulargewichtsmarker; 6: Serinmutante von MIF-Pep-16₁, 3.3 µM; 7: oxidiertes MIF-Pep-16₁, 3.3 µM; 8: reduziertes MIF-Pep-16₁, 3.3 µM.
Figur 5 stellt einen Zellproliferationstest in mikrosvaskulären Endothelzellen dar. Die Zugabe der MIFARs erfolgte wie im Balkendiagramm beschrieben.
Figur 6 stellt eine Coimmunpräzipitation (CoIP) von MIF-Pep-16₁ und der MPN-Domäne von JAB1 in vitro dar. 1 und 2, Überstände nach der Immunpräzipitation (Negativkontrollen); 3, CoIP von MIF-Pep-16₁ und der MPN-Domäne von JAB1; 4, Kontrolle: wie 3, aber Beads ohne Streptavidin.

Das Sequenzprotokoll ist Bestandteil dieser Lehre und stellt in den SEQ ID Nr. 1 bis SEQ ID Nr. 37 MIFARs dar (SEQ ID Nr. 1 bis SEQ ID Nr. 16, SEQ ID Nr. 19 bis SEQ ID Nr. 23 und SEQ ID Nr. 25 bis SEQ ID Nr. 37 sind nicht erfindungsgemäße MIFARs).

SEQ ID Nr. 1 zeigt die 115 Aminosäuren umfassende Aminosäuresequenz von Mono-MIF-Rekombein-1, wobei das Molekül gegenüber der Wildtyp-Sequenz an Position 49 einen Pro-Rest enthält.

SEQ ID Nr. 2 zeigt die 115 Aminosäuren umfassende Sequenz von Mono-MIF-Rekömbein-2, wobei das Molekül gegenüber der Wildtyp-Sequenz an den Positionen 49 und 108 Pro-Reste enthält.

SEQ ID Nr. 3 zeigt die 115 Aminosäuren umfassende Sequenz von Mono-MIF-Rekombein-3, wobei das Molekül gegenüber der Wildtyp-Sequenz an Position 108 einen Pro-Rest enthält.

SEQ ID Nr. 4 zeigt die 115 Aminosäuren umfassende Sequenz von Mono-MIF-Rekombein-4, wobei das Molekül gegenüber der Wildtyp-Sequenz an den Positionen 40 49 Pro-Reste enthält.

SEQ ID Nr. 5 zeigt die 115 Aminosäuren umfassende Sequenz von Mono-MIF-Rekombein-5, wobei das Molekül gegenüber der Wildtyp-Sequenz an den Positionen 97 und 108 Pro-Reste enthält.

SEQ ID Nr. 6 zeigt die 115 Aminosäuren umfassenden Sequenzen der Mono-MIF-Rekombeine 6 bis 8, wobei die Moleküle gegenüber der Wildtyp-Sequenz an den Positionen 41 und 46 substituiert sind.

SEQ ID Nr. 7 zeigt die 115 Aminosäuren umfassende Sequenz von Mono-MIF-Synthein-1, wobei das Molekül gegenüber der Wildtyp-Sequenz an Position 49 einen Nα-Ala-Rest enthält.

SEQ ID Nr. 8 zeigt die 115 Aminosäuren umfassende Sequenz von Mono-MIF-Synthein-2, wobei das Molekül gegenüber der Wildtyp-Sequenz an Position 49 einen Nα-Ala-Rest und an Position 108 einen Nα-Gly-Rest enthält.

SEQ ID Nr. 9 zeigt die 115 Aminosäuren umfassende Sequenz von Mono-MIF-Synthein-3, wobei das Molekül gegenüber der Wildtyp-Sequenz an Position 108 einen Nα-Gly-Rest enthält.

SEQ ID Nr. 10 zeigt die 115 Aminosäuren umfassende Sequenz von Mono-MIF-Synthein-4, wobei das Molekül gegenüber der Wildtyp-Sequenz an Position 40 einen Nα-Val-Rest und an Position 49 einen Nα-Ala-Rest enthält.

SEQ ID Nr. 11 zeigt die 115 Aminosäuren umfassende Sequenz von Mono-MIF-Synthein-5, wobei das Molekül gegenüber der Wildtyp-Sequenz an Position 97 einen Nα-Ile-Rest und an Position 108 einen Nα-Gly-Rest enthält.

SEQ ID Nr. 12 zeigt die 115 Aminosäuren umfassende Sequenz von MIF-Synthein-1, wobei das Molekül gegenüber der Wildtyp-Sequenz an den Positionen 57, 60 und 81 substituiert ist.

SEQ ID Nr. 13 zeigt die 115 Aminosäuren umfassenden Sequenzen der MIF-Cyclo-Syntheine 1 bis 16, wobei die Moleküle gegenüber der Wildtyp-Sequenz an den Positionen 56 und 61 substituiert sind.

SEQ ID Nr. 14 zeigt die 115 Aminosäuren umfassenden Sequenzen der MIF-Cyclo.-Syntheine 17 bis 24, wobei die Moleküle gegenüber der Wildtyp-Sequenz an den Positionen 55 und 62 substituiert sind.

SEQ ID Nr. 15 zeigt die 115 Aminosäuren umfassenden Sequenzen der MIF-Cyclo-Syntheine 25 bis 60, wobei die Moleküle gegenüber der Wildtyp-Sequenz an den Positionen 56, 57, 60 und 61 substituiert sind.

SEQ ID Nr. 16 zeigt die 115 Aminosäuren umfassenden Sequenzen der MIF-Cyclo-Syntheine 61 bis 96, wobei die Moleküle gegenüber der Wildtyp-Sequenz an den Positionen 55, 57, 60 und 62 substituiert sind.

SEQ ID Nr. 17 zeigt die 16 Aminosäuren umfassende Sequenz des MIF-Peptidfragmentes MIF-Pep-16₁.

SEQ ID Nr. 18 zeigt die 16 Aminosäuren umfassende Sequenz des MIF-Peptidfragmentes MIF-Pep-16₂.

SEQ ID Nr. 19 zeigt die 11 Aminosäuren umfassende Sequenz des MIF-Peptidfragmentes MIF-Pep-11.

SEQ ID Nr. 20 zeigt die 12 Aminosäuren umfassende Sequenz des MIF-Peptidfragmentes MIF-Pep-12.

SEQ ID Nr. 21 zeigt die 13 Aminosäuren umfassende Sequenz des MIF-Peptidfragmentes MIF-Pep-13.

SEQ ID Nr. 22 zeigt die 14 Aminosäuren umfassende Sequenz des MIF-Peptidfragmentes MIF-Pep-14.

SEQ ID Nr. 23 zeigt die 15 Aminosäuren umfassende Sequenz des MIF-Peptidfragmentes MIF-Pep-15.

SEQ ID Nr. 24 zeigt die 16 Aminosäuren umfassende Sequenz des MIF-Peptidfragmentes P16-Mut-1.

SEQ ID Nr. 25 zeigt die 115 Aminosäuren umfassende Sequenz von MIF-Triple-Mutein-1.

SEQ ID Nr. 26 zeigt die 115 Aminosäuren umfassende Sequenz von MIF-Triple-Mutein-2.

SEQ ID Nr. 27 zeigt die 115 Aminosäuren umfassende Sequenz von MIF-Triple-Mutein-3.

SEQ ID Nr. 28 zeigt die 115 Aminosäuren umfassende Sequenz von MIF-Triple-Mutein-4.

SEQ ID Nr. 29 zeigt die 115 Aminosäuren umfassende Sequenz von MIF-Triple-Mutein-5.

SEQ ID Nr. 30 zeigt die 115 Aminosäuren umfassende Sequenz von MIF-Triple-Chemoin-1.

SEQ ID Nr. 31 zeigt die 21 Aminosäuren umfassende Sequenz von β1/β4-Mini-MIF-1.

SEQ ID Nr. 32 zeigt die 22 Aminosäuren umfassende Sequenz von β1/β4-Mini-MIF-2.

SEQ ID Nr. 33 zeigt die 23 Aminosäuren umfassende Sequenz von β1/β4-Mini-MIF-3.

SEQ ID Nr. 34 zeigt die 22 Aminosäuren umfassende Sequenz von β1/β4-Mini-MIF-4.

SEQ ID Nr. 35 zeigt die 23 Aminosäuren umfassende Sequenz von β1/β4-Mini-MIF-5.

SEQ ID Nr. 36 zeigt die 24 Aminosäuren umfassende Sequenz von β1/β4-Mini-MIF-6.

SEQ ID Nr. 37 zeigt die 25 Aminosäuren umfassende Sequenz von β1/β4-Mini-MIF-7.

### Beispiel 1 (nicht erfindungsgemäß) Inhibitorische Wirkung von MIF-Triple-Chemoin-1

Humanes Wildtyp-MIF (wt MIF) wurde an allen drei Cysteinpositionen in einer Alkylierungsreaktion mit 4-Vinylpyridin chemisch modifiziert und die immunologische Aktivität dieses MIF-Triple-Chemoins-1 überprüft. Die Testung führte zu dem überraschenden Befund, dass solch dreifach modifiziertes MIF nicht aktivierend wirkte oder einfach inaktiv war, sondern zu einer substantiellen Inhibition des immunstimulierenden Effekts des im Zellwachstum bereits vorhandenen MIFs (des endogenen MIFs) führte. Die Hemmung ist in etwa 50% (netto) so effektiv wie die beobachtete Aktivierung durch eine hohe und optimale Zugabe rekombinanten MIFs. Daraus läßt sich schließen, dass dreifach chemisch modifiziertes MIF dieser Art einen biologischen Hemmstoff für die Wirkung natürlichen MIFs darstellt und somit als potentiell MIF-hemmendes Arzneimittel Einsatz finden kann. Rekombinante oder synthetische MIF-Mutanten, bei denen an allen drei CysteinPositionen natürliche Aminosäuren oder unnatürliche Aminosäuren - die strukturell der chemischen Modifikation aus dem MIF-Triple-Chemoin-1 ähneln - eingesetzt sind, weisen ähnliche beziehungsweise je nach Ausführung noch höhrere Hemmwirkung auf. Der Assay wurde wie in Vieth et al., 1994 und Mischke et al., 1997 beschrieben durchgeführt. Die Pufferkontrolle erfolgte mit 20 mM Natriumphosphatpuffer, pH-Wert 7,2.

### Beispiel 2 (erfindungsgemäß) Das MIF-Pep-16₁-Peptid als CXXC-disulfidbasierter Redoxkatalysator und hochpotenter kleinmolekularer MIF-Agonist

Um bei potentiellen MIF-basierten Anwendungen in der Therapie und Diagnostik die bekannten Nachteile, zum Beispiel schwierige Herstellung, Handhabungsprobleme, Abbaugefahr und Galenik und ähnliches, bei der Verwendung kompletter MIF-Proteinmoleküle zu umgehen, wurden kleine MIF-basierte Moleküle mit 11 bis 16 Aminosäuren, wie zum Beispiel MIF-Peptide, entwickelt. Eines dieser Peptide, MIF-Pep-16₁, welches der humanen MIF-Sequenz 50-65 entspricht und die Cysteinreste 57 und 60 überspannt, wurde nach Standardmethoden chemisch synthetisiert und untersucht. Bereits bei der analytischen Überprüfung der Synthese des Peptids wurde offenbar, dass sich solch konzipierte MIF-16er Peptide durch außerordentliche Reaktivität und Instabilität auszeichnen.

Oxidationsstudien mit anschließender HPLC-Analytik oder Untersuchungen durch Circulardichroismus-Spektropolarimetrie zeigten dann auch, dass MIF-Pep-16₁ eine starke Tendenz zur Ausbildung einer intramolekularen Disulfidbrücke hat. Dies zeigte sich durch die für Disulfidbrücken charakteristische Erhöhung des φ-Wertes von oxidiertem MIF-Pep-16₁ im Nah-UV-GD bei ∼275 nm sowie durch den ebenfalls charakteristischen Anstieg der β-Turn-Konformation (negativer Peak bei -225 nm im Fern-UV-CD). Dieser geht auf die durch das Disulfid induzierte Loopstabilisierung zurück, welche die entsprechende β-Turnstruktur stabilisiert. Parallel dazu wurde eine entsprechend verringerte random coil-Struktur bei oxidiertem MIF-Pep-16₁ gegenüber reduzietem MIF-Pep-16₁ beobachtet. Diese Daten zeigen eindeutig, dass MIF-Pep-16₁ trotz der geringen Molekülgröße eine Sekundärstruktur ausbildet und eine reaktive Dithiolstruktur enthält. Bei den durch CD analysierten Oxidationsstudien von MIF-Pep-16₁ wurde experimentell wie folgt vorgegangen: MIF-Pep-16₁ wurde entweder mit DTT reduziert oder durch Luftoxidation oxidiert (100 µM Peptid in 10 mM Tris-HCl, pH 8,0) beziehungsweise reduziertes MIF-Pep-16₁ wieder reoxidiert. Bei der Oxidation haben die Cystein-Thiole Gelegenheit, inter- oder intramolekulare Disulfide auszubilden. HPLC und LC-MS-Analysen zeigen, dass die gebildeten Disulfide zu über 90 % intramolekular waren. Dies ist Voraussetzung für die katalytische Disulfid-Redoxaktivität, wie sie erforderlich ist, wenn diese Peptide die MIF-artige Redoxaktivität aufweisen sollen. Anschließend wurde oxidiertes und reduziertes MIF-Pep-16₁ mit der Nah- und Fern-UV-CD-Methode verglichen. Die Daten rechtfertigen die Annahme, dass loopstabilisierte Varianten von MIF-Pep-16₁, wie zum Beispiel die Cyclo-MIF-Peps reaktiver und disulfidfreudiger sind, da solche Varianten der Disulfidausbildung im CXXC-Bereich wegen der durch den Loop erzwungenen Nähe noch reaktiver sein sollten. Des weiteren wurde untersucht, ob MIF-Pep-16₁ katalytische Redoxaktivität - wie für das MIF-Mutterprotein beschrieben - aufweisen. Dazu wurden die Peptide im Insulin-Reduktions-Assay (nach Holmgsen 1979; Kleemann et al., 1998) bei einer Konzentration von 40 µM getestet. Es zeigte sich, dass MIF-Pep-16₁ eine signifikante kataytische Aktivität (0,26 Units für wtMIF) aufweist. Diese Daten zeigen, dass MIF-Pep-16₁ eine enzymatische Redoxaktivität wie MIF aufweist und dass diese von der Anwesenheit der Cysteinreste beziehungsweise von der Ausbildung einer katalytischen Disulfidbrücke abhängig sind. Kleinmolekulare Peptide von der Art des MIF-Pep-16₁ können daher die enzymatische Aktivität von MIF simulieren und kommen als pharmakologische Agonisten für die Enzymwirkung von MIF in Frage. Dagegen stellen die Varianten mit inaktiven oder ausgetauschten Cysteinen potentielle Inhibitoren dar. Eine wichtige Frage war jedoch, ob MIF-Pep-16₁ oder verwandte kleine Peptide auch immunologische MIF-Aktivität zeigen. Zur Klärung wurden die Peptide im Glucocorticoid-Overriding-Assay (nach Calandra et al., 1995: 1 µg/ml LPS, 10⁻⁹ M Dex, 10 nM wt MIF, 600 nM MIF-Pep-16₁), einem für MIF spezifischen Macrophagen-Aktivierungstest untersucht, der darauf beruht, dass die durch Glucocorticoide supprimierte Immunanwort von Macrophagen durch MIF wieder aufgehoben oder antagonisiert wird. Da kein bis zum gegenwärtigen Zeitpunkt entdecktes anderes Cytokin diese Fähigkeit besitzt, kommt diesem Test eine herausragende Bedeutung bei der Evaluierung potentieller MIF-basierter Arzneimittel zu. Diese Ergebnisse zeigen, dass MIF-Pep-16₁ bei einer für kleine Peptide optimalen Konzentration von 0,6 µM beziehungsweise 6,6 µM die maximale MIF-Bioaktivität aufweist.

Diese Daten geben Hinweise darauf, dass MIF-Pep-16₁-artige MIF-Peptide potentielle MIF-basierte Therapeutika oder Diagnostika sind, während Varianten mit Austausch oder Modifikation der Cysteine potentielle biologische Hemmstoffe oder Antagonisten sind. Die Daten zeigen weiter, dass die jeweiligen Effekte durch chemische Loopstabilisierung (zum Beispiel Cyclisierung durch Lactamverbrückung) verstärkt werden können.

### Beispiel 3 Immobilisierung von MIF auf Oberflächen: Verfahren und Bioaktivität (erfindungsgemäß)

MIF-Pep-16₁ oder humanes rekombinantes MIF oder Mono-MIF-Rekombein-1 wurde biotinyliert und an einen mit Streptavidin funktionalisierten Siliciumwafer gebunden und die MIF-Immobilisierung durch grenzflächentechnische Analytik wie Ellipsometrie und Schichtdickenmessung bestätigt. So immobilisiertes MIF wurde dann auf seine Oxidoreduktaseaktivität untersucht. Dazu wurde ein definiertes Waferstück in ein Reaktionsgefäß eingebracht und dort mit der Reaktionslösung des HED-Oxidoreduktasetests in Kontakt gebracht (siehe z. B. Kleemann et al., 1998a). Nach 10 Minuten wurde diese Lösung wieder abpipettiert und die Menge an umgesetzten HEDs im Fotometer bestimmt. Immobilisiertes MIF zeigte 64+/-7 % Aktivität im Vergleich zur Aktivität von Wildtyp-MIF im Standardassay in Lösung. Wurde MIF kovalent an einen aminofunktionalisierten Wafer gebunden, wurden noch ca. 39 % Aktivität gemessen. Die Immobilisierung von MIF an CNBr-aktivierte Sepharose als Träger (nach Muronetz et al., 1988) war ebenfalls erfolgreich. So hatte immobilisiertes MIF 71+/-11 % Aktivität im Vergleich zur Kontrollmessung in Lösung.

Immobilisiertes MIF-Pep 16₁ hatte 33 +/- 9% Aktivität i.V. (im Vergleich) zum Peptid in Lösung und 12 +/- 3% Aktivität i.V. zu MIF.

Immobilisiertes MonoMIF-Rekombein-1 zeigte 65 +/-12% Aktivität i.V. zum Rekombein in Lösung und 92 +/- 4% i.V. zu MIF.

### Beispiel 4: Kompetition von MIF-Jab-Bindung durch MIFARs (erfindungsgemäß)

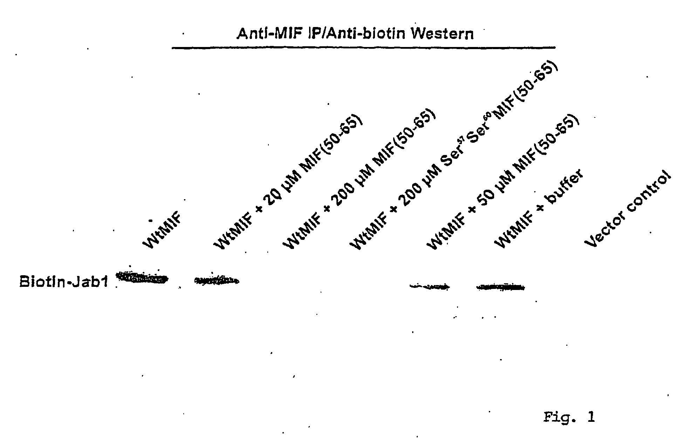

Es wurden in vitro-Studien in einem TNT-Retikulozytenlysatsystem durchgeführt. Die Figur 1 stellt einen Anti-Biotin-S-HRP Westernblot dar. MIF-Pep 16₁ (MIF 50 bis 65) kompetiert in konzentrationsabhängiger Weise mit dem Wildtyp-MIF für die Bindung mit Biotin markiertem Jab1 wie aus der Figur 1 ersichtlich. Gleiches gilt für das 16 Aminosäuren lange MIF-Derivat mit den Aminosäuren 50-65 von Wildtyp-MIF, wobei die Aminosäuren Cys57 und Cys60 durch Ser57 und Ser60 ausgetauscht sind.

### Beispiel 5 (erfindungsgemäß):

Immunologische MIF-artige Aktivität des MIF-Pep-16₁-Peptids in einem THP-1-Monozyten Zelltest und Applikation für Screeningverfahren zur Identifizierung MIF-Pep-16₁-Peptid-basierter kleinmolekularer Substanzen als Therapeutika.

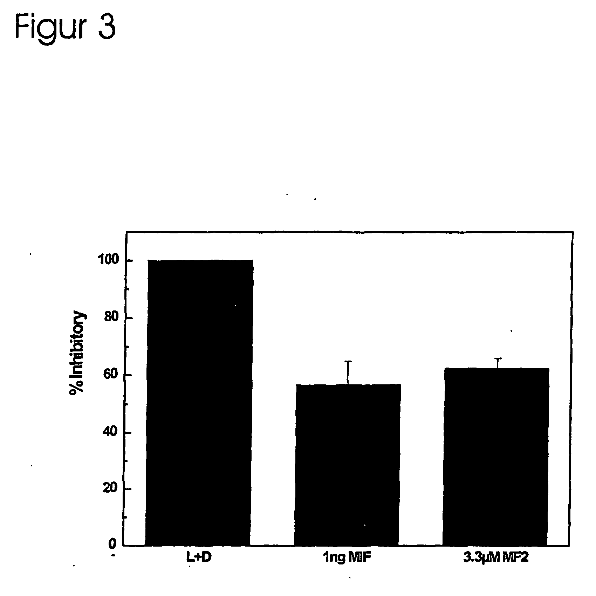

Das MIF-Pep-16₁-Peptid weist MIF-artige immunologische Aktivität im Glucocorticoid-Overriding-Test auf (siehe Beispiel 2). Der originär beschriebene in der Forschung eingesetzte Glucocorticoid-Overriding-Test in primären Blutmonozyten aus Spenderblut ist jedoch für pharmakologische Screeningtests ungeeignet, da der Test eine Spenderabhängige Variabilität aufweist und primäre Blutmonozyten nicht in den für Screeningansätze benötigten großen Mengen zur Verfügung stehen. Es wurde daher untersucht, ob die Glucocorticoid-Overriding-Aktivität von MIF-Pep-16₁-Peptid auch in einer für Screeningzwecke geeigneten Monozytenlinie gemessen werden kann. Hierzu wurde der Glucocorticoid-Overriding-Test zunächst in der humanen THP-1-Zelllinie etabliert. Hierzu mussten einige der Testparameter wie die Inkubationszeit und die Dexamethasonkonzentration neu adaptiert werden.
Der optimierte Test in THP-1-Zellen ist im Folgenden beschrieben:
Es werden THP-1-Zellen (human acute monocytic leukemia cell line) verwendet. Die Zellen werden in RPMI 1640+ 10% FCS; 2 mM L-Glutamine; 100 U/ml Pen/Strep kultiviert. Alle Lösungen zuzugebender Stoffe werden 100-fach konzentriert angesetzt, so dass im Assay je 5 µl pro well zugegeben werden können. Dexamethason wird in EtOH gelöst, dann in Medium weiterverdünnt. LPS wird vor der Verdünnung für 5 min ultrabeschallt. Je 5 x 10⁵ dieser Zellen werden in 0.5 ml Medium mit 1% FCS in 24-Well-Gewebekulturplatten, nach mindestens 3 hr (oder übernacht) absitzen, inkubiert (37°C, 5 % CO₂). Danach wird Dexamethason (Endkonzentration 10⁻⁶ M) und MIF (Endkonzentration 1 ng/ml) oder das MIF-Pep-16₁-Peptid zugegeben. Nach einer Stunde Inkubation wird 1 µg/ml LPS pro Well (Endkonz.) und wiederum MIF oder Peptid zugegeben und für 4 h inkubiert. Nach Zentrifugation des Überstandes zur Entfernung der Zellen wird die TNF-Konzentration mittels ELISA (R&D Systems, Wiesbaden) bestimmt. MIF weist in diesem Test gute Glucocorticoid-Overriding-Aktivität auf. Das MIF-Pep-16₁-Peptid weist ebenfalls gute Aktivität auf (siehe Figur 3); eine Serin-Mutante des MIF-Pep-16₁-Peptids, Ser57/Ser60- MIF-Pep-16₁, weist dagegen kaum Aktivität auf.

Der Test ist somit als Screeningtest zur Identifizierung kleinmolekularer MIF-Inhibitoren geeignet. MIF-Pep-16₁ beziehungsweise die Serinmutante sind als Ausgangsstrukturen (Template) zur Identifizierung solcher Inhibitoren geeignet.

### Beispiel 6 (erfindungsgemäß):

Das MIF-Pep-16₁-Peptid weist MIF-artige Aktivität hinsichtlich der Regulation von Zellzyklus- und Zellwachstumsvorgängen auf und ist zur Anwendung als Leitstruktur zur Identifizierung MIF-Pep-16₁-Peptid-basierter Substanzen als Therapeutika bei Zellzyklus-abhängigen Krankheiten wie Krebs, Immunschwächen oder anderen Zellwachstumsentgleisungen geeignet.

MIF reguliert über die Bindung an JAB1 zentrale Zellzyklusvorgänge (Kleemann et al., Nature 408, 2000). Es wurde überprüft, ob das MIF-Pep-16₁-Peptid ebenfalls solch eine zellregulatorische Aktivität aufweist. Hierzu wurde untersucht, ob MIF-Pep-16₁ die zellulären Konzentrationen des Zellzyklusinhibitors p27-Kip1 modulieren kann.
NIH 3T3-Fibroblasten wurden wie in Kleemann et al. (Nature 408, 2000) beschrieben kultiviert und induziert und mit MIF bzw. den MIFAR-Peptiden behandelt.

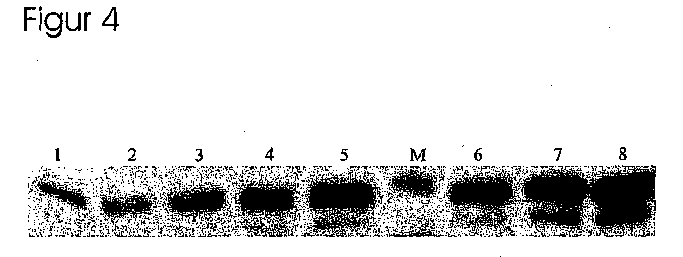

Figur 4 zeigt, dass das Peptid MIF-Pep-16₁ eine zu MIF vergleichbare p27-modulierende Zellaktivität aufweist und zwar vergleichbar in seiner oxidierten und reduzierten Form. Die Serinmutante von MIF-Pep-16₁ weist eine relativ schwächere Aktivität auf.

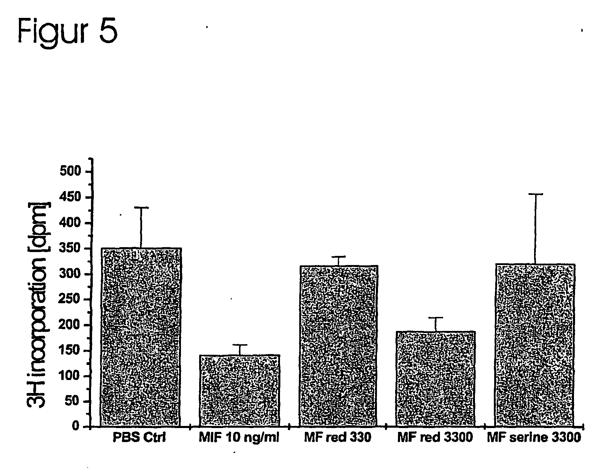

Figur 5 zeigt, dass ähnliche biologische Aktivitäten in einem Zellproliferationsassay in mikrovaskulären Endothelzellen beobachtet werden. In diesem Fall inhibieren MIF und MIF-Pep-16₁ das Zellwachstum, was mit der Induktion des Zellzyklusinhibitors p27 (siehe Figur 4) korrespondiert; die Serinmutante von MIF-Pep-16₁ zeigt keinen Effekt.

Aus den Ergebnissen ergibt sich, dass MIFARs wie MIF-Pep-16₁ als Antitumortherapeutika geeignet sind und/oder als Ausgangstrukturen zur Entwicklung verbesserter Antitumorstrukturen dienen können.

### Beispiel 7 (erfindungsgemäß):

Bindung von biotinyliertem MIF-Pep-16₁-Peptid an JAB1 und die JAB1-Domäne MPN

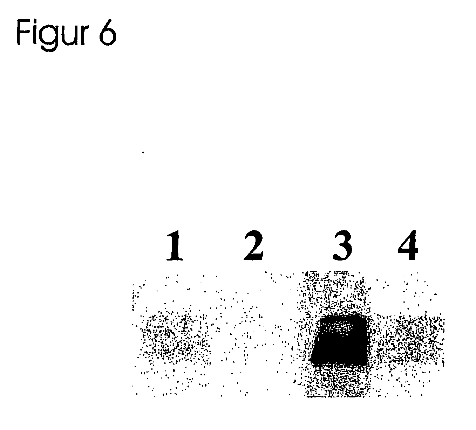

Analog zur Versuchsdurchführung in Beispiel 4 wurde hier die direkte Bindung von biotinyliertem MIF-Pep-16₁-Peptid an die MPN-Domäne von JAB1, welche die MIF-bindende Domäne von JAB1-darstellt, getestet. Figur 6 zeigt, dass biotinyliertes MIF-Pep-16₁-Peptid an MPN bindet.
Die Bindung wurde durch folgenden experimentellen Ansatz überprüft:
16 µl ³⁵S MPN wurden im in vitro-Translationskit der Firma RiNA mit 484 µl PBS-Puffer versetzt. Es wurden 8.3 µl biotinyliertes MIF-Pep-16₁-Peptid (Endkonzentration: 10 µM) zugegeben. Es wurde für 16 hr bei 4 Grad inkubiert. Dann wurden 600 µl Dynabeads M-280 (Dynal Streptavidin magnetic beads) zugegeben und für weitere 90 min bei Raumtemperatur inkubiert. Nach Zentrifugieren wurde der Überstand verworfen und die Beads in 60 µl Elektrophoresepuffer für 10 min bei 100°C gekocht. Die Proben wurden dann in einem 18%igen Gel aufgetrennt, das Gel für 2 hr getrocknet und die Radioaktivität exponiert.

Die Ergebnisse sind in Figur 6 dargestellt und zeigen Coimmunpräzipitation von der MPN-Domäne mit MI F-Pep-16₁.

Daraus lässt sich schließen, dass biotinylierte MIFARs in Biotin-Streptavidin-basierten Screeningassays (zum Beispiel unter Verwendung entsprechender Hochdurchsatz-Mikrotiterplattenassays) zur Identifizierung von MIF-basierten Therapeutika dienen können.

### Literatur

1. Bacher, M. et al., *Am. J*. *Pathol . (1997), 15,* 235-246
2. Bendrat, K. et al., *Biochemistry (1997), 36,* 15356 - 15362
3. Bernhagen, J. et al. , *J. Exp. Med. (1996) , 183,* 277 - 282
4. Bernhagen, J. et al., *Trends Microbiol. (1994), 2,* 198 - 201
5. Bernhagen, J. et al., *Nature (1993), 365,* 756 - 759
6. Bloom, B. & Bennett, B., *Science (1966), 153,* 80 - 82
7. Bozza, M. et al., *J. Exp. Med. (1999), 189,* 341 - 346
8. Calandra, T. et al., *Nature (1995), 377*, 68 - 71
9. Calandra, T. et al., *Nat . Med. (2000), 6,* 164 - 169
10. David J.R. et al., *Proc. Natl. Acad. Sci. (1966), 56,* 72 - 77
11. Donnelly, S. C. et al., *Nat. Med. (1997), 3,* 320 - 323
12. Galat, A., et al., *Eur. J. Biochem. (1994), 224,* 417 - 421
13. Gossen, M. und Bujard, H., *PNAS 89 (1992),* 5547 - 5551
14. Hermanowski-Vosatka, A. et al., *Biochemisty (1999), 38,* 12841 - 12849
15. Holmgren, A., *J. Biol. Chem. (1979)*, *254,* 9627 - 9632
16. Kleemann, R. et al., *J.. Mol.Biol. (1998a), 280,* 85 -102
17. Kleemann, R. et al., *Eur. Biochem. (1999), 28,* 753 - 766
18. Kleemann, R. et al., *FEBS Lett. (19898b), 430,* 191 - 196
19. Lan, H. Y. et al., *J. Exp. Med. (1997)*, *185*, 1455 - 1465
20. Leech, M. et al., *Arthritis & Rheum. (1998), 41*, 910 - 917
21. Mikulowska, A. et al., *J. Immunol.* (1997), *158,* 5514 - 5517
22. Mischke, R. et al., *FEBS Lett.* (1997), 414, 226 - 232
23. Rosengren, E. et al., *Mol. Med. (1996),* 2, 143 - 149
24. Rosengren, E. et al., *FEBS Lett. (1997), 417,* 85 - 88
25. Sambrook, J. et al., *Molecular Cloning, A Laboratory Manual, Cold Spring Harbor Laboratory Press,* 2. *Ausgabe 1989*
26. Swope, M. et al., *EMBO J*. *(1998), 1*, 3534 - 3541.
27. Sun, H.-W. et al., *Proc. Natl. Acad. Sci. USA (1996), 93,* 5191 - 5196
28. Vieth, M. et al., *Scan. J*. *Immunol. (1994),* 40, 403 - 409
28. Weiser, W. Y. et al., *Proc. Natl. Acad. Sci. USA (1989), 86,* 7522 - 7526

## Patentansprüche

1. Derivat des MIFs, wobei das Derivat aus 16 Aminosäuren besteht und ausgewählt ist aus der Gruppe bestehend aus
a) einem Derivat des MIFs, wobei das Derivat die Aminosäuresequenz 50-65 oder 51-66 des MIFs aufweist und
b) einem Derivat des MIFs, wobei das Derivat die Aminosäuresequenz 50-65 oder 51-66 des MIFs aufweist und 1 bis 5 Aminosäuren des Derivats ausgetauscht sind.

2. Nucleinsäuresequenz, die ein Derivat des MIFs nach Anspruch 1 codiert.

3. Nucleinsäuresequenz nach Anspruch 2, die eine DNA-Sequenz ist.

4. Nucleinsäuresequenz nach Anspruch 2, die eine RNA-Sequenz ist.

5. Vektor, der eine Nucleinsäuresequenz nach einem der Ansprüche 2 bis 4 enthält.

6. Vektor nach Anspruch 5, der ein viraler Vektor und/oder ein Plasmid ist.

7. Vektor nach einem der Ansprüche 5 oder 6, wobei die Nucleinsäuresequenz mit regulatorischen Elementen operativ verknüpft ist.

8. Wirtszelle, die mit einem Vektor nach einem der Ansprüche 5 bis 7 transformiert ist oder von solch einer Zelle abstammt.

9. Wirtszelle nach Anspruch 8, wobei die Wirtszelle procaryotisch oder eucaryotisch ist.

10. Zellkultur, die mindestens eine Wirtszelle nach Anspruch 8 oder 9 enthält.

11. Zellkultur nach Anspruch 10, wobei die Wirtszelle in der Lage ist, ein Derivat des MIFs nach Anspruch 1 zu produzieren.

12. Verfahren zur Herstellung eines Derivats des MIFs nach Anspruch 1, wobei eine Wirtszelle nach einem der Ansprüche 8 oder 9 in einem Kulturmedium unter Bedingungen kultiviert wird, die eine Bildung des Derivats erlauben und das Derivat gewonnen wird.

13. Verfahren zur Herstellung eines Derivats des MIFs nach Anspruch 1, wobei das Derivat chemisch hergestellt wird.

14. Derivat nach Anspruch 1, wobei das Derivat immobilisiert ist.

15. Derivat nach Anspruch 1 oder 14, wobei das, vorzugsweise immobilisierte, Derivat in medizinischen Vorrichtungen, drug delivery-Vorrichtungen, Matrizen für drug-screening-Bibliotheken und/oder in einer applizierten Katalyse-Vorrichtung vorhanden ist.

16. Antikörper, der eine spezifische Affinität zu einem Derivat nach Anspruch 1 aufweist.

17. Antikörper, der eine Affinität zu dem Antikörper nach Anspruch 16 hat.

18. Antikörper nach einem der Ansprüche 16 oder 17, der modifiziert ist.

19. Arzneimittel, **gekennzeichnet durch** einen Gehalt an einem Derivat des MIF nach Anspruch 1, einer Nucleinsäure nach einem der Ansprüche 2 bis 4, einem Vektor nach einem der Ansprüche 5 bis 7, einer Wirtzelle nach Anspruch 8 und/oder 9, einer Zellkultur nach Anspruch 10 und/oder 11 und/oder eines Antikörpers nach einem der Ansprüche 16 bis 18, gegebenenfalls zusammen mit einem pharmazeutisch akzeptablen Träger.

20. Verwendung eines Derivats des MIFs nach Anspruch 1, einer Nucleinsäure nach einem der Ansprüche 2 bis 4, einem Vektor nach einem der Ansprüche 5 bis 7, einer Wirtzelle nach Anspruch 8 und/oder 9, einer Zellkultur nach Anspruch 10 und/oder 11 und/oder eines Antikörpers nach einem der Ansprüche 16 bis 18 zur Herstellung eines Arzneimittels oder Diagnosemittels zur Diagnostik oder Therapie von Krebserkrankungen, inflammatorischen Krankheiten, einer Krankheit des Respirationstraktes und/oder einer Krankheit des Zentralnervensystems.

21. Verfahren zum Identifizieren von pharmazeutisch aktiven Substanzen, wobei ein Derivat des MIFs nach Anspruch 1 zusammen mit Jab in An- und Abwesenheit einer zu untersuchenden Substanz inkubiert, die Interaktion zwischen dem Derivat des MIFs und Jab in Abhängigkeit von der An- und Abwesenheit der zu testenden Substanz bestimmt und so eine die Interaktion von MIF mit Jab modulierende und daher pharmazeutisch aktive Substanz identifiziert wird.

22. Verfahren nach Anspruch 21, wobei das Derivat von MIF, Jab und die zu untersuchende Substanz zusammen mit MIF inkubiert und die Interaktion von Jab mit MIF in Abhängigkeit von der An- und Abwesenheit der zu untersuchenden Substanz bestimmt wird.

## Claims

1. A derivative of the MIF, wherein the derivative consists of 16 amino acids and is selected from the group consisting of
a) a derivative of the MIF wherein the derivative comprises the amino acid sequence 50-65 or 51-66 of the MIF and
b) a derivative of the MIF wherein the derivative comprises the amino acid sequence 50-65 or 51-66 of the MIF and 1 to 5 amino acids of the derivative are substituted.

2. A nucleic acid sequence which codes for a derivative of the MIF according to claim 1.

3. A nucleic acid sequence according to claim 2 which is a DNA sequence.

4. A nucleic acid sequence according to claim 2 which is an RNA sequence.

5. A vector which contains a nucleic acid sequence according to any of claims 2 to 4.

6. A vector according to claim 5, which is a viral vector and/or a plasmid.

7. A vector according to either of claims 5 and 6, wherein the nucleic acid sequence is operatively combined with regulating elements.

8. A host cell which is transformed with a vector according to any of claims 5 to 7 or stems from such a cell.

9. A host cell according to claim 8, wherein the host cell is prokaryotic or eukaryotic.

10. A cell culture which contains at least one host cell according to claim 8 or 9.

11. A cell culture according to claim 10, wherein the host cell is in a position to produce a derivative of the MIF according to claim 1.

12. A method of producing a derivative of the MIF according to claim 1, wherein a host cell according to either of claims 8 and 9 is cultivated in a culture medium under conditions which allow formation of the derivative and the derivative is recovered.

13. A method of producing a derivative of the MIF according to claim 1, wherein the derivative is produced chemically.

14. A derivative according to claim 1, wherein the derivative is immobilised.

15. A derivative according to claim 1 or 14, wherein the derivative, preferably immobilised, is present in medicinal devices, drug delivery devices, matrices for drug screening libraries and/or in an applied catalysis apparatus.

16. An antibody which has a specific affinity for a derivative according to claim 1.

17. An antibody which has an affinity to an antibody according to claim 16.

18. An antibody according to either of claims 16 and 17, which is modified.

19. A medicament, **characterized by** a content of a derivative of the MIF according to claim 1, a nucleic acid according to any of claims 2 to 4, a vector according to any of claims 5 to 7, a host cell according to claim 8 and/or 9, a cell culture according to claim 10 and/or 11, and/or an antibody according to any of claims 16 to 18, optionally together with a pharmaceutically acceptable carrier.

20. Use of a derivative of the MIF according to claim 1, a nucleic acid according to any of claims 2 to 4, a vector according to any of claims 5 to 7, a host cell according to claim 8 and/or 9, a cell culture according to claim 10 and/or 11, and/or an antibody according to any of claims 16 to 18 for producing a medicament or diagnostic agent for diagnosis or therapy of cancer illnesses, inflammatory illnesses, an illness of the respiratory tract and/or an illness of the central nervous system.

21. A method of identifying pharmaceutically active substances, wherein a derivative of the MIF according to claim 1 is incubated together with Jab in the presence and in the absence of a substance to be investigated, the interaction between the derivative of the MIF and Jab is determined in the presence and in the absence of the substance to be tested and a substance modulating the interaction of MIF with Jab and therefore pharmaceutically active is thus identified.

22. A method according to claim 21, wherein the derivative of MIF, Jab and the substance to be investigated is incubated together with MIF and the interaction of Jab with MIF is determined in dependence on the presence and in the absence of the substance to be investigated.

## Revendications

1. Dérivé du MIF, composé de 16 acides aminés et choisi parmi le groupe composé de
a) un dérivé du MIF, présentant la séquence d'acides aminés 50-65 ou 51-66 du MIF et
b) un dérivé du MIF, présentant la séquence d'acides aminés 50-65 ou 51-66 du MIF, et dont 1 à 5 acides aminés du dérivé sont remplacés.

2. Séquence d'acides nucléiques, qui code pour un dérivé du MIF selon la revendication 1.

3. Séquence d'acides nucléiques selon la revendication 2, qui est une séquence d'ADN.

4. Séquence d'acides nucléiques selon la revendication 2, qui est une séquence d'ARN.

5. Vecteur, qui contient une séquence d'acides nucléiques selon l'une quelconque des revendications 2 à 4.

6. Vecteur selon la revendication 5, qui est un vecteur viral et/ou un plasmide.

7. Vecteur selon l'une quelconque des revendications 5 ou 6, dans lequel la séquence d'acides nucléiques est associée de manière opérationnelle à des éléments régulateurs.

8. Cellule hôte, qui est transformée avec un vecteur selon l'une quelconque des revendications 5 à 7 ou qui descend d'une telle cellule.

9. Cellule hôte selon la revendication 8, dans laquelle la cellule hôte est procaryote ou eucaryote.

10. Culture cellulaire, qui contient au moins une cellule hôte selon la revendication 8 ou 9.

11. Culture cellulaire selon la revendication 10, dans laquelle la cellule hôte est en mesure de produire un dérivé du MIF selon la revendication 1.

12. Procédé de fabrication d'un dérivé du MIF selon la revendication 1, dans lequel une cellule hôte selon l'une quelconque des revendications 8 ou 9 est cultivée dans un milieu de culture sous des conditions qui permettent la formation du dérivé, et dans lequel le dérivé est obtenu.

13. Procédé pour la fabrication d'un dérivé du MIF selon la revendication 1, dans lequel le dérivé est fabriqué chimiquement.

14. Dérivé selon la revendication 1, dans lequel le dérivé est immobilisé.

15. Dérivé selon la revendication 1 ou 14, dans lequel le dérivé, de préférence immobilisé, est présent dans des dispositifs médicaux, des dispositifs de libération de médicaments, des matrices pour des bibliothèques de criblage de médicaments et/ou dans un dispositif de catalyse appliquée.

16. Anticorps, qui présente une affinité spécifique pour un dérivé selon la revendication 1.

17. Anticorps, qui a une affinité pour l'anticorps selon la revendication 16.

18. Anticorps selon l'une quelconque des revendications 16 ou 17, qui est modifié.

19. Médicament,
**caractérisé par**
une teneur en un dérivé du MIF selon la revendication 1, un acide nucléique selon l'une quelconque des revendications 2 à 4, un vecteur selon l'une quelconque des revendications 5 à 7, une cellule hôte selon la revendication 8 et/ou 9, une culture cellulaire selon la revendication 10 et/ou 11 et/ou un anticorps selon l'une quelconque des revendications 16 à 18, le cas échéant tous avec un porteur pharmaceutiquement acceptable.

20. Utilisation d'un dérivé du MIF selon la revendication 1, d'un acide nucléique selon l'une quelconque des revendications 2 à 4, d'un vecteur selon l'une quelconque des revendications 5 à 7, d'une cellule hôte selon la revendication 8 et/ou 9, d'une culture cellulaire selon la revendication 10 et/ou 11 et/ou d'un anticorps selon l'une quelconque des revendications 16 à 18 pour la fabrication d'un médicament ou d'un produit diagnostique pour le diagnostic ou le traitement de pathologies cancéreuses, de maladies inflammatoires, d'une maladie du tractus respiratoire et/ou d'une maladie du système nerveux central.

21. Procédé pour l'identification de substances pharmaceutiquement actives, dans lequel un dérivé du MIF selon la revendication 1 est mis en incubation avec Jab en présence ou en l'absence d'une substance à examiner, l'interaction entre le dérivé du MIF et Jab est définie en fonction de la présence ou de l'absence de la substance à examiner et ainsi une substance modulant l'interaction du MIF et de Jab et par conséquent pharmaceutiquement active est identifiée.

22. Procédé selon la revendication 21, dans lequel le dérivé du MIF, Jab et la substance à examiner sont mis en incubation avec le MIF et l'interaction de Jab et du MIF est déterminée en fonction de la présence ou de l'absence de la substance à examiner.
